# EUROPEAN PATENT SPECIFICATION

(11) **EP 4 262 950 B1**
(45) Date of publication and mention of the grant of the patent: **05.08.2026**
(21) Application number: 21907207.1
(22) Date of filing: 24.08.2021
(51) Int. Cl.: A61M 16/08

(54) **A RESPIRATORY CONNECTOR ASSEMBLY AND RESPIRATORY SUPPORT SYSTEM**
ATEMVERBINDERANORDNUNG UND ATEMUNTERSTÜTZUNGSSYSTEM
ENSEMBLE RACCORD RESPIRATOIRE ET SYSTÈME DE SOUTIEN RESPIRATOIRE

(30) Priority: 18.12.2020 US 202063127682 P
(43) Date of publication of application: 25.10.2023
(60) Divisional application: 26154611.3
(73) Proprietor: Fisher & Paykel Healthcare Limited, East Tamaki, Auckland 2013 (NZ)
(72) Inventor: MCDERMOTT, Gareth Thomas, Auckland, 2013 (NZ); EVANS, Alicia Jerram Hunter, Auckland, 2013 (NZ); HOW, David-Reyner Cailun, Auckland, 2013 (NZ); WALMSLEY, Jaiden Gray, Auckland, 2013 (NZ); OSBORNE, Hamish Adrian, Auckland, 2013 (NZ); EDWARDS, Taylor James, Auckland, 2013 (NZ); BURGESS, Aidan Robert, Auckland, 2013 (NZ); ROBB, Adrian David James, Auckland, 2013 (NZ)
(74) Representative: Ström & Gulliksson AB
(86) International application number: PCT/NZ2021/050143
(87) International publication number: WO 2022/131931

(56) References cited:
- EP-A1- 2 108 927
- WO-A1-2018/033863
- WO-A1-2019/211152
- WO-A1-2020/157707
- CN-A- 108 025 153
- US-A1- 2008 072 903
- US-A1- 2010 282 253
- US-A1- 2020 360 637

## Description

### FIELD OF THE INVENTION

The present disclosure generally relates to devices and methods for providing breathable gases to a user. In particular, certain features, aspects and advantages of the present disclosure relate to apparatuses and techniques that provide for or enable connections between components of a gas supply system. More particularly, the present disclosure relates to a connector and connector assembly for altering a property of a gas flow. The disclosure also relates to a respiratory support system and tube assembly having a selected resistance to flow.

### BACKGROUND

Respiratory gas supply systems provide for the delivery of breathable gas to a patient. Respiratory gas supply systems typically include a fluid connection between a gas supply such as a flow generator to the airway of the patient. Such systems include a number of different components to ensure gas is correctly delivered to a patient. Some of the components may be single use components that are disposed of after each patient use, while other components may be multi-use components.

WO2019/211152A1 describes a respiratory gas flow coupling. EP2108927A1 describes a patient ventilation system and, more particularly, to a bi-directional flow sensor measuring respiratory flow to and from a patient. CN108025153A describes a respiratory therapy appliance comprising a respiratory air channel having a constriction with a variable cross section.

In some situations, it is necessary to connect single use components to multi-use components. Single use components may typically be used for patient interfacing components, whereas more complex components, such as pressure relief valves, humidifiers, and flow generators may be used for multiple patients.

It is desirable to be able to configure a respiratory circuit to accommodate the varying requirements of different patients. For example, different patients may have different fit requirements for a patient interface which may mean different patients require different types or sizes of a patient interface. It is also desirable to provide substantially consistent respiratory support to a patient across various respiratory systems. For example, a patient may need to transit between various parts of a medical facility and the patient is provided with a patient interface that is single patient use and which would follow the patient during such transit. Therefore when interchanging components in a circuit to customise the circuit (for example where the patient interface has to connect to a different respiratory device resulting from the patient transit), there may be differences in the properties of the interchanged components, which include but are not limited to the length of conduits, the physical flexibility of components of the circuit, and other components (e.g. humidification apparatus) of the respiratory circuit which may vary depending on the subject or the therapy or treatment required. These components could be single or multi-patient use components. These different interchangeable components, may exhibit different flow characteristics in use, for example the pressure drop across the components may be different.

Generally, a system that is tuned to provide breathable gas at a given pressure, flow rate, temperature, and/or humidity, for a given component such as a given user interface, may not provide the breathable gas as desired if a different, and particularly, an unintended, user interface is substituted. In systems containing pressure sensitive components such as the flow compensated pressure relief valve described in WO2020157707, utilizing unintended components could result in resistances to flow in that system that may not be appropriate and may therefore compromise the safety and/or effectiveness of the system.

Further, in some scenarios, it is desirable to be able to move a patient between multiple respiratory systems without removing the patient interface from the patient. That is, a patient interface may stay attached to a patient, while the flow source providing a respiratory support is changed. For example, during a medical procedure and/or during such a procedure where the patient may become apnoeic, a nasal cannula may be used with a patient and a first respiratory system. The patient may then be moved to a second respiratory system in other situations where respiratory support is provided.

The different respiratory systems the patient is moved between may have different resistance to flow and driving pressure requirements. Components such as user interface components that are suitable for a first system may not be suitable for use in the second system, limiting the ability to move a patient between systems without removing the patient interface and replacing it with one that the second system or component(s) of the second system is/are configured for.

While prior arrangements have provided the desired therapies, a need remains for further improvements to respiratory therapy systems and methods relating to the same. Accordingly, it is an object of at least preferred embodiments of the present invention to address one or more of the above-mentioned disadvantages and/or to at least provide the public with a useful alternative.

In this specification where reference has been made to patent specifications, other external documents, or other sources of information, this is generally to provide a context for discussing features of the invention. Unless specifically stated otherwise, reference to such external documents or sources of information is not to be construed as an admission that such documents or such sources of information, in any jurisdiction, are prior art or form part of the common general knowledge in the art.

### SUMMARY OF THE INVENTION

The invention is defined by the appended claims.

Described herein is a connector assembly for use in a respiratory support system, comprising:
a first connector; a second connector for connection to the first connector to thereby define a gas flow path through the connectors; and
a flow modifying arrangement having a first part and a second part; wherein the first connector comprises the first part of the flow modifying arrangement, and the second connector comprises the second part of the flow modifying arrangement; and wherein the flow modifying arrangement is configured to change a property of a gas flow along the gas flow path when the first and second connectors are connected. The first part of the flow modifying arrangement and the second part of the flow modifying arrangement together define a tortuous flow path through the flow modifying arrangement.

In some forms, the property of a gas flow along the gas flow path is one or more of: pressure, turbulence, velocity.

In some forms, the first and second parts of the flow modifying arrangement are disposed in the gas flow path.

In some forms, wherein the first and second parts of the flow modifying arrangement together define a high resistance portion of the flow path having a higher resistance to flow than portions of the flow path immediately upstream and downstream of the flow modifying arrangement. Preferably, the first and second parts forming a dominant flow restriction in a respiratory system. Optionally, the dominant flow restriction decouples the upstream and downstream portions.

The first and second parts of the flow modifying arrangement together define a tortuous flow path through the flow modifying arrangement.

In some forms, wherein the flow modifying arrangement is configured to increase the velocity of gas flow along the gas flow path at the flow modifying arrangement compared to the velocity of gas flow along the gas the flow path upstream and/or downstream of the flow modifying arrangement.

In some forms, the flow modifying arrangement is configured to substantially change the pressure along the gas flow path.

In some forms, the first and second parts of the flow modifying arrangement are arranged co-axially.

In some forms, the first part of the flow modifying arrangement is proximate to the second part.

In some forms, the first part of the flow modifying arrangement is spaced from the second part, and wherein the size of the spacing is selected to provide a pre-determined change to a property of gas flow along the gas path.

In some forms, the first part of the flow modifying arrangement is upstream or downstream of the second part, with respect to the gas flow path.

In some forms, the first and second parts of the flow modifying arrangement define a high resistance flow path through the flow modifying arrangement, the flow path having a cross sectional area that is selected to provide a pre-determined change to a property of gas flow across the flow modifying arrangement.

In some forms, one or both of the first and/or second parts of the flow modifying arrangement is integral with the respective first or second connector.

In some forms, the first part of the flow modifying arrangement comprises one or more of: a constriction or an obstruction, orifice, venturi, or a blocking member; and wherein the second part of the flow modifying arrangement comprises one or more of: a constriction or an obstruction, orifice, venturi, or a blocking member

In some forms, the first part of the flow modifying arrangement comprises a constriction or an obstruction. Preferably, the obstruction is a partial flow obstruction. Preferably, the first part of the flow modifying arrangement comprises one or more of a blocking member, a constricted portion, and/or one or more bars. Preferably, the first part of the flow modifying arrangement comprises at least one blocking member that is substantially cylindrical, frustoconical, cuboidal, pyramidal, prismatic, conical, or spherical. Preferably, the blocking member comprises a longitudinal axis that is substantially parallel to a longitudinal axis of the first connector. Preferably, the blocking member is positioned in the gas flow path, with the blocking member longitudinal axis generally co-linear with the longitudinal axis of the first connector.

Optionally, the first part of the flow modifying arrangement further comprises an elongate positioning portion for positioning the blocking member in the flow. In some forms, the blocking member comprises a cylindrical plate attached to the elongate positioning portion. Preferably, the cylindrical plate has a greater radial diameter than that of the elongate positioning portion.

In some forms, the first part of the flow modifying arrangement comprises one or more bars. Preferably, the, or each, bar extends across at least a portion of the flow path.

In some forms, the, or each, bar is a crossbar arranged substantially transverse to the flow path. Optionally, the, or each, bar has a cross-section that is substantially circular, quadrilateral, triangular, elliptical, cross-shaped, tear-drop shaped or biconvex.

In some forms, the second part of the flow modifying arrangement comprises a constriction or an obstruction. Preferably, the second part of the flow modifying arrangement comprises a constriction, the constriction defining a portion of the connector assembly having a reduced internal diameter along the gas flow path. Preferably, the constriction is formed from a thickened portion of a wall of the connector.

In some forms, the first part of the flow modifying arrangement comprises an obstruction and the second part of flow modifying arrangement comprises a constriction, and wherein the first part is arranged proximate to the second part to define a tortuous gas flow path through the flow modifying arrangement. In some forms, the second part of the flow modifying arrangement comprises an orifice. Preferably, the orifice comprises an orifice plate.

In some forms, the first part of the flow modifying arrangement comprises a blocking member and the second part of flow modifying arrangement comprises an orifice, and wherein the first part is arranged proximate to the second part to define a tortuous gas flow path through the flow modifying arrangement. Preferably, the size of the blocking member and/or the size of the orifice is selected to provide a desired resistance to flow through the flow modifying portion.

In some forms, the second part of the flow modifying arrangement comprises an annular venturi. Optionally, the profile of the venturi is curved. Alternatively, the profile of the venturi comprises one or more linear portions.

In some forms, the first part of the flow modifying arrangement comprises one or more bars and the second part of the flow modifying arrangement comprises a venturi; wherein the profile of the venturi comprises one or more linear portions; and
wherein the, or each, bar is arranged proximate to the venturi to define a tortuous gas flow path through the flow modifying arrangement. Preferably, the venturi is asymmetrical about a transverse plane at a throat of the venturi, and wherein the throat is arranged proximate the bar(s).

In some forms, the dimensions and/or shape of the bar(s) and/or dimensions and/or shape of the venturi is selected to provide a desired change in a flow property across the flow modifying arrangement.

In some forms, the second part of the flow modifying arrangement comprises one or more bars. Preferably, the bar(s) of the first part of the flow modifying arrangement is/are oriented in a different direction compared to the bar(s) of the second part of the flow modifying arrangement. Optionally, the bar(s) of the first part of the flow modifying arrangement are of a different thickness compared to the bar(s) of the second part of the flow modifying arrangement. Optionally, the bar(s) of the second part of the flow modifying arrangement extend(s) substantially transverse to the gas flow path in a different orientation compared to the bar(s) of the first part of the flow modifying arrangement.

In some forms, the, or each, bar has a cross-section that is substantially circular, quadrilateral, triangular, elliptical, cross-shaped, tear-drop shaped or biconvex.

In some forms, the first part of the flow modifying arrangement causes only a minimal, insignificant or negligible change to the selected property of a gas flow across the first connector, when the first connector is separate from the second connector.

In some forms, the first part of the flow modifying arrangement does not substantially alter a property of a gas flow across the first connector, when the first connector is separate from the second connector.

In some forms, wherein the second part of the flow modifying arrangement causes only a minimal, insignificant or negligible change to the selected property of a gas flow across the second connector, when the second connector is separate from the first connector.

In some forms, the second part of the flow modifying arrangement does not substantially alter a property of a gas flow across the second connector, when the second connector is separate from the first connector.

In some forms, the first connector is releasably engageable with the second connector. Optionally, the first connector comprises a first engagement feature, and the second connector comprises a second, complementary engagement feature. In some forms, the first and second connectors comprise alignment features, to orientate the first part of the flow modifying arrangement relative to the second part of the flow modifying arrangement.

In some forms, wherein the first connector is arranged upstream of the second connector.

In some forms, the first connector is coupled to an inspiratory conduit or filter.

In some forms, the second connector is coupled to a patient interface conduit or filter.

In some forms, the flow modifying arrangement is configured for use in a high flow type respiratory support system. The flow modifying arrangement may be configured for use at flow rates of more than 20 L/min. Alternatively, the flow modifying arrangement may be configured for use at flow rates of 20-90L/min. Preferably, the flow modifying arrangement is configured for use at flow rates of 40-70L/min.

In some forms, the connector assembly is arranged along a circuit of the respiratory system.

Also described herein is a connector for use in a respiratory support system, the connector defining a gas flow path through the connector, and comprising a first part of a flow modifying arrangement, wherein the connector is configured to engage with a second connector having a second part of the flow modifying arrangement, to form an assembly that is configured to change a property of a gas flow along the gas flow path through the assembly.

The above-described connector is for use in the above-described connector assembly.

In some forms, the first part of the flow modifying arrangement is configured to define a high resistance portion of the flow path when arranged in line with and proximal to the second part of the flow modifying arrangement.

In some forms, the first part of the flow modifying arrangement is configured to define a torturous flow path through the flow modifying arrangement when arranged in line with and proximal to the second part of the flow modifying arrangement.

In some forms, the flow modifying arrangement is configured to substantially change the pressure along the gas flow path.

In some forms, the first part of the flow modifying arrangement comprises a constriction or an obstruction. Preferably, the obstruction is a partial flow obstruction. Preferably, wherein the first part of the flow modifying arrangement comprises one or more of a blocking member, a constricted portion, and/or one or more bars. Preferably, the first part of the flow modifying arrangement comprises at least one blocking member that is substantially cylindrical, frustoconical, cuboidal, pyramidal, prismatic, conical, or spherical.

In some forms, the blocking member comprises a longitudinal axis that is substantially parallel to a longitudinal axis of the first connector. Preferably, the blocking member is positioned in the gas flow path, with the blocking member longitudinal axis generally co-linear with the longitudinal axis of the first connector.

In some forms, the first part of the flow modifying arrangement comprises one or more bars. Preferably, the, or each, bar extends across at least a portion of the flow path. Preferably, the, or each, bar is a crossbar arranged substantially transverse to the flow path.

In some forms, the, or each, bar has a cross-section that is substantially circular, quadrilateral, triangular, elliptical, cross-shaped, tear-drop shaped or biconvex.

In some forms, the first part of the flow modifying arrangement comprises a constriction, the constriction defining a portion of the connector assembly having a reduced internal diameter along the gas flow path. Optionally, the constriction is formed from a thickened portion of a wall of the connector.

In some forms, the first part of the flow modifying arrangement comprises an orifice. Preferably, the orifice comprises an orifice plate.

In some forms, the first part of the flow modifying arrangement comprises an annular venturi. Preferably, the profile of the venturi is curved. Alternatively, the profile of the venturi comprises one or more linear portions. Optionally, the venturi is asymmetrical about a transverse plane at a throat of the venturi, and wherein the throat is arranged proximate the bar(s).

In some forms, the first part of the flow modifying arrangement is configured to interact with a second part of the flow modifying arrangement that is different in form or orientation to the first part of the flow modifying arrangement. Preferably, the first part of the flow modifying arrangement causes only a minimal, insignificant or negligible change to the selected property of a gas flow across the first connector, when the first connector is separate from the second connector. Preferably, the first part of the flow modifying arrangement does not substantially alter a property of a gas flow across the first connector, when the first connector is separate from the second connector. Preferably, the connector does not substantially alter a property of a gas flow when the first connector is engaged with a third connector that does not contain a flow modifying portion.

In some forms, wherein the first connector comprises an engagement feature for engaging with the second connector. Preferably, the first connector comprises an alignment feature for orientating the first connector relative to the second connector.

Also described herein is a patient interface assembly comprising a connector described above, and a patient interface.

In some forms, the patient interface is a non-sealing patient interface. Preferably, the patient interface is nasal cannula.

In some forms, the connector is attached to the end of a conduit.

Also described herein is a tube assembly comprising a conduit, a first connector configured to couple to a humidifier and/or a flow source, provided at a first end of the conduit; a second connector, as described above, provided at a second end of the conduit.

In some forms, wherein the conduit comprises one or more heating elements.

In some forms, the conduit comprises one or more sensing elements.

Also described herein is a respiratory support system comprising: a humidifier; an inspiratory conduit in fluid communication with the humidifier; a patient interface; and a connector as described above, provided at an end of the conduit.

In some forms, the respiratory support system further comprises a flow compensated pressure relief valve in fluid communication with the inspiratory conduit.

In some forms, the respiratory support system further comprises a flow source. Preferably, the flow source generates a high-volume gas flow.

In some forms, the conduit comprises one or more heating elements.

In some forms, the conduit comprises one or more sensing elements.

In some forms, the patient interface is a non-sealing patient interface. Preferably, wherein the patient interface is nasal cannula.

In a **first aspect,** the disclosure relates to a respiratory support system comprising a flow source, and a respiratory circuit comprising a pressure relief valve and a tube assembly in fluid communication with the flow source and pressure relief valve, wherein the respiratory circuit downstream of the pressure relief valve has a resistance to gas flow of 30 - 50 cmH2O at a selected gas flow rate.

In some forms, the selected gas flow rate is 70 L/min.

In some forms, the pressure relief valve is a flow compensated pressure relief valve.

In some forms, the pressure relief valve operates at flow rates between about 30 and about 70 litres per minute.

In some forms, the pressure relief valve comprises a sensing member arranged to sense a flow rate through the system. Preferably, the sensing member is configured to dynamically change a pressure threshold of the pressure relief valve in use.

In some forms, the respiratory circuit further comprises a humidification chamber and a conduit fluidly connected between the flow source and the humidification chamber.

In some forms, the circuit further comprises a patient interface, wherein the patient interface comprises a non-sealing patient interface and wherein the non-sealing patient interface is a nasal cannula.

In some forms, the circuit further comprises a filter.

In some forms, the tube assembly is an inspiratory conduit. Preferably, wherein the inspiratory conduit is fluidly connected to a patient interface.

In some forms, the inspiratory conduit has a length of between 1 to 3 m. Preferably, the inspiratory conduit has a length of between 1.5 to 2.5 m. Preferably, the inspiratory conduit has a length of about 2.4 m.

In some forms, the respiratory circuit comprises a flow disrupting feature. Optionally, the tube assembly comprises a flow disrupting feature. Preferably, the flow disrupting feature comprises a flow obstruction or a construction

In some forms, the inspiratory conduit has an end connector, and the flow disrupting feature is provided in the end connector.

In some forms, the tube assembly has an end connector, and the flow disrupting feature comprises an orifice. Preferably, the orifice has an inner diameter of about 7.1 mm to about 7.3 mm. Preferably, the orifice has an inner diameter of about 7.2 mm.

In some forms, the inspiratory conduit defines a flow path and comprises a heating wire disposed in the flow path. Preferably, the heating wire has the form of a double helix.

In some forms, the heating wire has an electrical resistance of 11.4 - 12.7 ohm/metre at room temperature.

In some forms, the inspiratory conduit comprises a corrugated tube, and wherein the corrugations have an outer diameter of between about 23mm and about 25mm, an inner diameter of between about 20 mm and about 21 mm, and a pitch of about 4.5 mm.

In some forms, the system is a high flow system wherein the flow source is configured to provide a gases flow rate of more than 20 L/min. Preferably, the gas flow is humidified.

In some forms, the flow source is configured to provide a gases flow rate of 20-90L/min. Preferably, the flow source is configured to provide a gases flow rate of 40-70L/min.

In some forms, the respiratory circuit has a resistance to gas flow of 35 - 45 cmH2O at a gas flow rate of 70 L/min. Preferably, the respiratory circuit has a resistance to gas flow of about 40 cmH2O at a gas flow rate of 70 L/min.

Described herein is a tube assembly for use in a respiratory support system, comprising: a fluid conduit defining a flow path; and one or more end connectors; wherein the tube assembly comprises a flow disrupting feature to alter resistance to flow along the flow path, and wherein the tube assembly has a resistance to gas flow of more than to zero cmH2O to about 30 cm H2O at a selected gas flow rate.

In some forms, the selected gas flow rate is 90 L/min. Alternatively, the selected gas flow rate is 70 L/min. the selected gas flow rate is 40 L/min. Alternatively, the selected gas flow rate is 30 L/min. Alternatively, the selected gas flow rate is 20 L/min.

In some forms, the tube assembly is configured to be in fluid communication with a pressure relief valve. Preferably, the pressure relief valve is a flow compensated pressure relief valve. In some forms, the tube assembly is configured to the tube assembly is configured to couple (directly or indirectly) to the pressure relief valve upstream of the tube inlet.

In some forms, the one or more end connector(s) is integral with the fluid conduit.

In some forms, the fluid conduit has a length of between 1 to 3 m. Preferably, the inspiratory conduit has a length of more than 1.5 and less than or equal to about 2.5 m. Preferably, the inspiratory conduit has a length of between 2 m to 2.5 m. Preferably, the inspiratory conduit has a length of about 2.4 m.

In some forms, the flow disrupting feature comprises a flow obstruction or a constriction in the flow path.

In some forms, the fluid conduit has an end connector, and the flow disrupting feature is provided in the end connector. Preferably, the flow disrupting feature comprises an orifice. Optionally, the orifice has an inner diameter of about 7.0 mm to about 7.5 mm. Preferably, the orifice has an inner diameter of about 7.2 mm.

In some forms, the tube assembly further comprises a heating wire disposed in the flow path. Preferably, at least a portion of the heating wire is wound in a double helical manner.

In some forms, the resistance to flow of the tube assembly is between about 0.5 and about 25 cmH2O for a dry, non-humidified gases flow at a flow rate of between about 20 L/min and about 90 L/min.

In some forms, the resistance to flow of the tube assembly is between about 18 and about 25 cmH2O, for a non-humidified gases flow at a flow rate of 90 L/min. Preferably, the resistance to flow of the tube assembly is about 21 cmH2O for a non-humidified gases flow at a flow rate of 90 L/min

In some forms, the resistance to flow of the tube assembly is between about 20 and about 28 cmH2O, for a humidified gases flow at a flow rate of 90 L/min. Preferably, the resistance to flow of the tube assembly is about 24 cmH2O for a humidified gases flow at a flow rate of 90 L/min.

In some forms, the resistance to flow of the tube assembly is between about 11 and about 16 cmH2O, for a non-humidified gases flow at a flow rate of 70 L/min. Preferably, the resistance to flow of the tube assembly is about 13 cmH2O for a non-humidified gases flow at a flow rate of 70 L/min

In some forms, the resistance to flow of the tube assembly is between about 0.5 and about 28 cmH2O for a humidified gases flow at a flow rate of between about 20 L/min and about 90 L/min.

In some forms, the resistance to flow of the tube assembly is between about 12 and about 17 cmH2O, for a humidified gases flow at a flow rate of 70 L/min. Preferably, the resistance to flow of the tube assembly is about 15 cmH2O for a humidified gases flow at a flow rate of 70 L/min.

In some forms, wherein the resistance to flow of the tube assembly is between about 3.5 and about 5.5 cmH2O, for a non-humidified gases flow at a flow rate of 40 L/min. Preferably, the resistance to flow of the tube assembly is about 4.5 cmH2O for a non-humidified gases flow at a flow rate of 40 L/min

In some forms, wherein the resistance to flow of the tube assembly is between about 4 and about 6 cmH2O, for a humidified gases flow at a flow rate of 40 L/min. Preferably, the resistance to flow of the tube assembly is about 5 cmH2O for a humidified gases flow at a flow rate of 40 L/min.

In some forms, the resistance to flow of the tube assembly is between about 2 and about 3 cmH2O, for a non-humidified gases flow at a flow rate of 30 L/min. Preferably, the resistance to flow of the tube assembly is about 2.5 cmH2O for a non-humidified gases flow at a flow rate of 30 L/min

In some forms, the resistance to flow of the tube assembly is between about 2 and about 3.5 cmH2O, for a humidified gases flow at a flow rate of 30 L/min. Preferably, the resistance to flow of the tube assembly is about 3 cmH2O for a humidified gases flow at a flow rate of 30 L/min.

In some forms, wherein the resistance to flow of the tube assembly is between about 0.5 and about 1.5 cmH2O, for a non-humidified gases flow at a flow rate of 20 L/min. Preferably, the resistance to flow of the tube assembly is about 1 cmH2O for a non-humidified gases flow at a flow rate of 20 L/min

In some forms, the resistance to flow of the tube assembly is between about 0.5 and about 1.5 cmH2O, for a humidified gases flow at a flow rate of 20 L/min. Preferably, the resistance to flow of the tube assembly is about 1 cmH2O for a humidified gases flow at a flow rate of 20 L/min.

Also described herein is a kit of parts comprising: the tube assembly described above, and one or more of: a humidification chamber; a conduit for use upstream of a humidifier; a patient interface; and/or a filter.

In some forms, the tube assembly is configured for use as an inspiratory conduit.

In some forms, the kit further comprises a chamber for a humidifier and a dry-line conduit for use upstream of a humidifier.

In some forms, the kit further comprises a patient interface, wherein the patient interface comprises a non-sealing patient interface and wherein the non-sealing patient interface is a nasal cannula.

In some forms, the kit is provided in sealed, sterile packaging.

This disclosure may also be said broadly to consist in the parts, elements and features referred to or indicated in the specification of the application, individually or collectively, and any or all combinations of any two or more said parts, elements or features. Where specific integers are mentioned herein which have known equivalents in the art to which this disclosure relates, such known equivalents are deemed to be incorporated herein as if individually described.

The term 'comprising' as used in this specification and claims means 'consisting at least in part of'. When interpreting statements in this specification and claims that include the term 'comprising', other features besides those prefaced by this term can also be present. Related terms such as 'comprise' and 'comprised' are to be interpreted in a similar manner.

It is intended that reference to a range of numbers disclosed herein (for example, 1 to 10) also incorporates reference to all rational numbers within that range and any range of rational numbers within that range (for example, 1 to 6, 1.5 to 5.5 and 3.1 to 10). Therefore, all subranges of all ranges expressly disclosed herein are hereby expressly disclosed.

As used herein the term '(s)' following a noun means the plural and/or singular form of that noun. As used herein the term 'and/or' means 'and' or 'or', or where the context allows, both.

### BRIEF DESCRIPTION OF THE DRAWINGS

The present invention will now be described by way of example only and with reference to the accompanying drawings in which:
Figure 1 is a schematic illustrating an exemplary respiratory system;
Figure 2 is a graph illustrating the resistance to flow for various different sized cannulas;
Figure 3 is a side section view of a portion of one embodiment respiratory connector assembly;
Figure 4 is a side section view of the portion of a respiratory connector assembly shown in Figure 3, illustrating the flow path around a flow modifying arrangement;
Figure 5 is a schematic partial side perspective view of an example embodiment of a flow modifying arrangement;
Figure 6 is a side section view of the embodiment shown in Figure 5;
Figure 7 is a partial side perspective view of the example embodiment of a flow modifying arrangement shown in Figure 5, illustrating the flow path around the flow modifying arrangement;
Figure 8 is a side section view of the example embodiment of a flow modifying arrangement shown in Figure 5, illustrating the flow path through the flow modifying arrangement;
Figure 9 is a partial side perspective view of the first part of a flow modifying arrangement of Figures 5-8 but connected to a regular conduit, illustrating the flow path around the first part of the flow modifying arrangement;
Figure 10 is a side view corresponding to the view of Figure 9;
Figure 11 is a partial side perspective view of the second part of a flow modifying arrangement of Figures 5-8 but connected to a regular conduit, illustrating the flow path around the second part of the flow modifying arrangement;
Figure 12 is a side section view corresponding to the view of Figure 11;
Figure 13 is a side section view of a portion of a second embodiment respiratory connector assembly;
Figure 14 is a side section view of the portion of a respiratory connector assembly shown in Figure 13, illustrating the flow path through the flow modifying arrangement;
Figure 15 is a side section view of a third embodiment respiratory connector assembly;
Figure 16 is a side section view of the respiratory connector assembly shown in Figure 15, illustrating the flow path through a flow modifying arrangement;
Figure 17 is a side section view of a respiratory connector assembly according to a further embodiment;
Figure 18 is a side section view of the respiratory connector assembly shown in Figure 17, illustrating the flow path through the flow modifying arrangement;
Figure 19 is a side section view of a portion of a respiratory connector assembly according to another embodiment;
Figure 20 is a side section view of the respiratory connector assembly shown in Figure 19, illustrating the flow path through a flow modifying arrangement;
Figure 21 is a side section view of a portion of a respiratory connector assembly according to another embodiment;
Figure 22 is a side section view of through respiratory connector assembly shown in Figure 21, illustrating the flow path around a flow modifying arrangement;
Figure 23 is a side section view of a respiratory connector assembly according to another embodiment;
Figure 24 is a side section view of the respiratory connector assembly shown in Figure 23, illustrating the flow path through a flow modifying arrangement;
Figure 25 is a side section view of a portion of a respiratory connector assembly according to another embodiment;
Figure 26 is a side section view of the respiratory connector assembly shown in Figure 25, illustrating a first flow path through the flow modifying arrangement;
Figure 27 is a side section view of the portion of a respiratory connector assembly shown in Figure 25, illustrating a second flow path through the flow modifying arrangement;
Figure 28a is a schematic partial side perspective view of another example embodiment of a flow modifying arrangement in which the first and second parts of the flow modifying arrangement each comprise shaped flow obstructions;
Figure 28b is a schematic end view of the flow modifying arrangement shown in Figure 28a;
Figure 29a is a schematic side section view of another example embodiment of a flow modifying arrangement in which the first part of the flow modifying arrangement comprises a shaped flow obstruction, and the second part of the flow modifying arrangement comprises an orifice plate;
Figure 29b is a partial side perspective view of the flow modifying arrangement shown in Figure 29a;
Figure 29c is an end view of the flow modifying arrangement shown in Figure 29a;
Figure 30a is a schematic side perspective view of another example embodiment of a flow modifying arrangement in which the first and second parts of the flow modifying arrangement each comprise shaped flow obstructions;
Figure 30b is a front section view of the flow modifying arrangement shown in Figure 30a;
Figure 31a is a schematic side perspective view of a further example embodiment of a flow modifying arrangement in which the first and second parts of the flow modifying arrangement each comprise a plurality of bars;
Figure 31b is an end view of the flow modifying arrangement shown in Figure 30a;
Figure 32 is a partial side view of one embodiment of a tube assembly described herein for use in a respiratory system;
Figure 33 is a partial top elevation view of a heater wire for use in the tube assembly of Figure 32;
Figure 34 is a side elevation view corresponding to Figure 33;
Figure 35 is a cutaway perspective view of the heater wire of Figures 33 and 34;
Figure 36 is an end view of the connector end of the heater wire of Figures 33 to 35;
Figure 37 is a perspective view of an end connector for the tube assembly of Figure 32;
Figure 38 is an end elevation view of the end connector of Figure 37;
Figure 39 is a side section view taken along line F39 of Figure 38;
Figure 40 is a perspective view of the section of Figure 39;
Figure 41 is a schematic illustrating a further exemplary respiratory system;
Figure 42 is side section view of the tube assembly having a heater wire clip, showing the tube assembly of Figure 32 connected to upstream and downstream components; and
Figure 43 is a schematic illustrating a further exemplary respiratory system.

In these figures, like reference numbers are used for different embodiments to indicate like features, but with the addition of a multiple of 100.

### DETAILED DESCRIPTION OF A PREFERRED EMBODIMENT

### Introduction/system

A preferred form of respiratory connector assembly 2 is described below with reference to its use in a respiratory therapy system 10. It should be noted that the respiratory connector assembly 2 can be used with any suitable system that provides a gases stream from a gases source to a patient in use. For example, it could be used as part of a system to provide supplementary oxygen to a user, with the oxygen provided from a source such as a gas bottle or wall outlet. However, it is described below by example for use in a system that provides a heated, humidified, gases stream to a patient or user. The respiratory connector assembly 2 is suitable for use in the home or in a hospital environment.

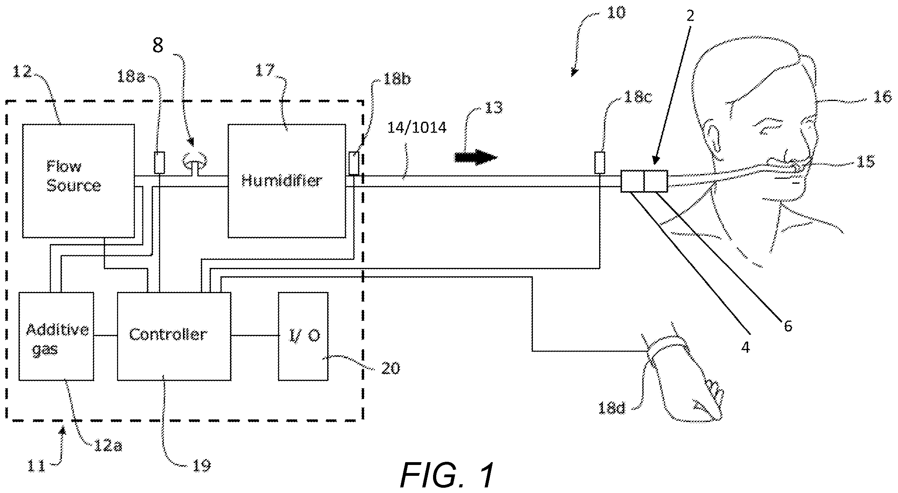

Referring to Figure 1, an exemplary respiratory therapy system 10 such as might be used with a preferred embodiment of respiratory connector assembly 2 is shown. The system 10 may be suitable for use in areas of a medical facility or environment where it is desirable to have medical devices and/or components 11 configured for multiple patient use.

A connector assembly 2 according to embodiments described herein is particularly adapted for use in respiratory systems such as CPAP or high flow respiratory gas systems, for example a high flow system for use in anaesthesia procedures. Respiratory systems in which the connector assembly 2 may be particularly useful are CPAP, BiPAP, high flow respiratory support, varying high flow respiratory support, low flow air delivery, low flow O₂ delivery, bubble CPAP, apnoeic high flow respiratory support (i.e. high flow to anesthetized patients), invasive ventilation and non-invasive ventilation systems. The connector assembly 2 described herein is particularly adapted for use in systems containing a pressure relief or regulating device. Further, a connector assembly 2 as described herein may be useful in systems other than respiratory systems.

In this specification, "high flow" means, without limitation, any gas flow with a flow rate that is higher than usual/normal, such as higher than the normal inspiration flow rate of a healthy patient. Alternatively or additionally, it can be higher than some other threshold flow rate that is relevant to the context - for example, where providing a gas flow to a patient at a flow rate to meet inspiratory demand, that flow rate might be deemed "high flow" as it is higher than a nominal flow rate that might have otherwise been provided. "High flow" is therefore context dependent, and what constitutes "high flow" depends on many factors such as the health state of the patient, type of procedure/therapy/support being provided, the nature of the patient (big, small, adult child) and the like. Those skilled in the art know from context what constitutes "high flow". It is a magnitude of flow rate that is over and above a flow rate that might otherwise be provided.

But, without limitation, some indicative values of high flow can be as follows.
- In some configurations, delivery of gases to a patient at a flow rate of greater than or equal to about 5 or 10 litres per minute (5 or 10 LPM or L/min).
- In some configurations, delivery of gases to a patient at a flow rate of about 5 or 10 LPM to about 150 LPM, or about 15 LPM to about 95 LPM, or about 20 LPM to about 90 LPM, or about 25 LPM to about 85 LPM, or about 30 LPM to about 80 LPM, or about 35 LPM to about 75 LPM, or about 40 LPM to about 70 LPM, or about 45 LPM to about 65 LPM, or about 50 LPM to about 60 LPM. For example, according to those various embodiments and configurations described herein, a flow rate of gases supplied or provided to an interface via a system or from a flow source, may comprise, but is not limited to, flows of at least about 5, 10, 20, 30, 40, 50, 60, 70, 80, 90, 100, 110, 120, 130, 140, 150 LPM, or more, and useful ranges may be selected to be any of these values (for example, about 20 LPM to about 90 LPM, about 40 LPM to about 70 LPM, about 40 LPM to about 80 LPM, about 50 LPM to about 80 LPM, about 60 LPM to about 80 LPM, about 70 LPM to about 100 LPM, about 70 LPM to about 80 LPM).

In "high flow" the gas delivered will be chosen depending on the intended use of the respiratory support. Gases delivered may comprise a percentage of oxygen. In some configurations, the percentage of oxygen in the gases delivered may be about 15% to about 100%, 20% to about 100%, or about 30% to about 100%, or about 40% to about 100%, or about 50% to about 100%, or about 60% to about 100%, or about 70% to about 100%, or about 80% to about 100%, or about 90% to about 100%, or about 100%, or 100%.

Flow rates for "High flow" for premature/infants/paediatrics (with body mass in the range of about 1 to about 30 kg) can be different. The flow rate can be set to about 0.4 L/min/kg to about 8 L/min/kg with a minimum of about 0.5 L/min and a maximum of about 25 L/min. For patients under 2 kg maximum flow may be set to 8 L/min.

High flow has been found effective in meeting or exceeding the patient's normal real inspiratory demand, to increase oxygenation of the patient and/or reduce the work of breathing. Additionally, high flow may generate a flushing effect in the nasopharynx such that the anatomical dead space of the upper airways is flushed by the high incoming gas flows. This creates a reservoir of fresh gas available of each and every breath, while minimising rebreathing of carbon dioxide, nitrogen, etc.

With reference to Figure 1, the system 10 may comprise an integrated or separate component based arrangement, generally shown in the dotted box 11 in Figure 1. In some configurations, the system 10 could comprise a modular arrangement of components. Hereinafter the system 10 will be referred to as the system, but this should not be considered limiting. The system 10 may include a flow source 12, such as an in-wall source of oxygen, an oxygen tank, a blower, a flow therapy apparatus, or any other source of oxygen or other gas. The system 10 may also comprise an additive gas source 12a, comprising one or more other gases that can be combined with the flow source 12.

The flow source 12 can provide a pressurised high gas flow 13 that can be delivered to a patient 16 via a inspiratory conduit 14, and patient interface 15 (such as a nasal cannula). A controller 19 controls the flow source 12 and additive gas source 12a through valves or the like to control flow and other characteristics such as any one or more of pressure, composition, concentration, volume of the high flow gas 13. A humidifier 17 is also optionally provided, which can humidify the gas under control of the controller and control the temperature of the gas. In some configurations the humidifier may be optional, or it may be preferred due to the advantages of humidified gases helping to maintain the condition of the airways. Humidification is preferably used with high flow gas flows to increase patient comfort, compliance, support and and/or safety. One or more sensors 18a, 18b, 18c, 18d, such as flow, oxygen, pressure, humidity, temperature or other sensors may be placed throughout the system and/or at, on or near the patient 16. The sensors may include a pulse oximeter 18d on the patient for determining the oxygen concentration in the blood.

The controller 19 may be coupled to the flow source 12, the additive gas source 12a, humidifier 17 and sensors 18a-18d. The controller 19 can operate the flow source to provide the delivered flow of gas. It can control the flow, pressure, composition (where more than one gas is being provided), volume and/or other parameters of gas provided by the flow source based on feedback from sensors. The controller 19 can also control any other suitable parameters of the flow source to meet oxygenation requirements. The controller 19 can also control the humidifier 17 based on feedback from the sensors 18a-18d. Using input from the sensors, the controller can determine oxygenation requirements and control parameters of the flow source 12 and/or humidifier 17 as required. An input/output (I/O) interface 20 (such as a display and/or input device) is provided. The input device is for receiving information from a user (e.g. clinician or patient) that can be used for determining, for example, oxygenation requirements. In some embodiments, the system may be without a controller and/or I/O interface. A medical professional such as a nurse or technician may provide the necessary control function.

The pressure may also be controlled. As noted above, the high gas flow (optionally humidified) can be delivered to the patient 16 via a inspiratory conduit 14 and the patient interface 15. The interface 15 may be any suitable respiratory patient interface, such as a nasal cannula, a mask, nasal interface, oral device or combination thereof. In some embodiments, the high gas flow (optionally humidified) can be delivered to the patient 16 for surgical uses, e.g. surgical insufflation. In these embodiments, the 'interface' could be a surgical cannula, trocar, or other suitable interface. The patient interface can be substantially sealed, partially sealed or substantially non-sealing. A nasal interface as used herein is a device such as a cannula, a nasal mask, nasal pillows, or other type of nasal device or combinations thereof. A nasal interface can also be used in combination with a mask or oral device (such as a tube inserted into the mouth) and/or a mask or oral device (such as a tube inserted into the mouth) that can be detached and/or attached to the nasal interface. A nasal cannula is a nasal interface that includes one or more prongs that are configured to be inserted into a patient's nasal passages. A mask refers to an interface that covers a patient's nasal passages and/or mouth and can also include devices in which portions of the mask that cover the patient's mouth are removable. A patient interface may also be an invasive patient interface such as a laryngeal mask airway or endotracheal tube. A patient interface may also be a nasal interface that includes nasal pillows that create a substantial seal with the patient's nostrils. The controller controls the system to provide the required oxygenation.

In some embodiments the system 10 may not require the use of a humidifier-that is, the flow of gas could be dry and unheated if required. Various types of therapy can be delivered by using the nasal interface. Generally any gases and respiratory gases delivery system can be used with the nasal interface. The respiratory humidification described here is just an example of the type of therapy and system the nasal interface can be used with or as part of.

In the exemplary system as shown in Figure 1 however, the humidifier 17 receives gases from a flow source 12, the gases becoming heated and humidified as they pass through the humidification chamber of humidifier 17. It should be noted that as outlined above, the flow source 12 could be replaced or supplemented by a wall port or a gas bottle. The flow source could be a gas bottle, a gas blender, a venturi device or a standard blower unit, or any other suitable system or device that supplies a gases stream. Humidified gases flow from the humidifier 17 through the inspiratory conduit 14 to the patient 16 by way of the patient interface 15.

In the embodiment shown, the patient interface 15 is a non-sealing nasal cannula or other suitable non-sealing or sealing patient interface, and the flow source is typically arranged to provide a high flow gas flow to the patient of more than 20 L/min, or more specifically, between 20-90 L/min, or more specifically between 40-70L/min. Different flow rates may be used for different patients. For example, lower flow rates may be more suitable for infants. The patient interface and flow source may also be used to provide high flow gas to the patient. However, it will be appreciated that alternative non-sealing or sealing patient interfaces such as oral or full-face masks may be used, and such alternative patient interfaces may require different gas flow rates. The patient interface may include a filter, or a filter may be provided separately for connection to the patient interface between the interface and the inspiratory conduit 14. The patient interface 15 may be provided with a patient interface conduit which supplies flow to the patient interface. The patient interface conduit may connect between the patient interface 15 and the inspiratory conduit 14.

With reference to Figure 1, the respiratory circuit of the respiratory therapy system 10 comprises the components from or between the flow source 12 and the patient 16 or at least a certain number of these components. For example, the respiratory circuit may comprise any of the following components: the humidifier 17, the humidification chamber of the humidifier 17, the inspiratory conduit 14, the patient interface 15, any conduit(s) upstream of the humidifier, filters, and any other conduits such as patient interface conduits. The respiratory circuit may define a flow path between the flow source 12 and the patient 16.

In some embodiments, the respiratory therapy system or circuit may comprise a pressure relief valve 8. The pressure relief valve 8 is typically positioned upstream of the humidifier 17 and conduit 14. The pressure relief valve 8 may dynamically adjust the pressure relief threshold with respect to the flow of gases through the valve. One such flow compensated pressure relief valve is described in PCT/IB2020/05076.

In a typical respiratory therapy system or circuit 10, the pressure relief valve 8 in the circuit may be tuned for use with a particular downstream resistance to flow / pressure drop. For example, it may be tuned for the resistance to flow of all downstream components, such as a specific patient interface 15 and/or conduit 14, and so may not operate effectively or correctly if the downstream resistance to flow significantly changes, for example by substituting a different sized cannula or patient interface with a different resistance to flow.

A pressure relief valve is usually provided as a safety feature so ineffective operation may be a safety risk, for example, it may lead to a risk of overpressure injury to the patient, or ineffective flow delivery to the patient, underdelivering flow.

A connector assembly 2 is provided for coupling two components of the respiratory circuit, for example, attaching the inspiratory conduit 14 to the patient interface or patient interface assembly 15, or for attaching the conduit 14 to the humidifier 17. The connector assembly 2 comprises a first connector 4, and a second connector 6. The first connector 4 and second connector 6 are located within the respiratory circuit. For example, the conduit 14 may comprise a first connector for removably engaging with a second connector attached to a patient interface, a conduit leading to the patient interface, or another component upstream of the patient interface in the respiratory system such as a filter. In another example, the humidifier or humidification chamber may comprise a first connector and the inspiratory tube 14 that connects to the humidification chamber may comprise the second connector. The second connector 6 connects to the first connector 4 to thereby define a gas flow path through the connectors.

The connector assembly 2 comprises a flow modifying arrangement having a first part and a second part. The first connector 4 comprises the first part of the flow modifying arrangement, and the second connector 6 comprises the second part of the flow modifying arrangement. The flow modifying arrangement is configured to change a property of a gas flow along the gas flow path.

More specifically, the connector assembly 2 described herein operates to modify the resistance to flow in the respiratory circuit and thereby allow pressure/flow sensitive components such as a pressure relief valve 8 to be used in a circuit with a cannula or patient interface 15 that would otherwise (without a flow modifying arrangement) have a different resistance to flow (RTF) that may not be compatible with said pressure/flow sensitive components. The flow delivered to a patient by the flow source 12 is based on an expected resistance to flow in the respiratory circuit, including the patient interface. In a circuit without a flow modifying arrangement, different sizes of patient interfaces having different resistances to flow would affect the flow delivered to the patient by the flow source. In a circuit with a flow modifying arrangement of the present invention, the respiratory circuit is modified by the connector assembly so that, for example, a circuit changed to have a different size of patient interface has a substantially similar resistance to flow, or a resistance to flow that is at or near an expected patient interface resistance to flow.

The resistance to flow of a particular embodiment of connector assembly 2 will be a function of the physical properties of each of the flow altering members of the connector. The resistance of a particular embodiment may also depend on one or more of the features of the respiratory circuit, the inspiratory conduit, or the respiratory system, including the number of exhaust openings, the position of any exhaust openings, the shape of the flow path formed by conduit, the cross-sectional shape and/or size of the conduit, the length of the flow path formed by conduit, and/or other physical properties of the particular embodiment of patient interface.

Resistance to flow is an additive property. As such, the overall resistance to flow of the respiratory circuit shown in Figure 1 can be determined from an aggregation of the resistances of each of the individual components. For example, for the embodiment shown in Figure 1, the overall resistance to flow of the respiratory circuit may be determined as the sum of the first resistances of the conduit 14, the patient interface 15, and the connector 2, including the first flow altering member, and the second flow altering member, as well as any other components that form part of the flow path in the respiratory circuit such as the patient interface conduit, or conduits upstream of the humidifier or humidification chamber.

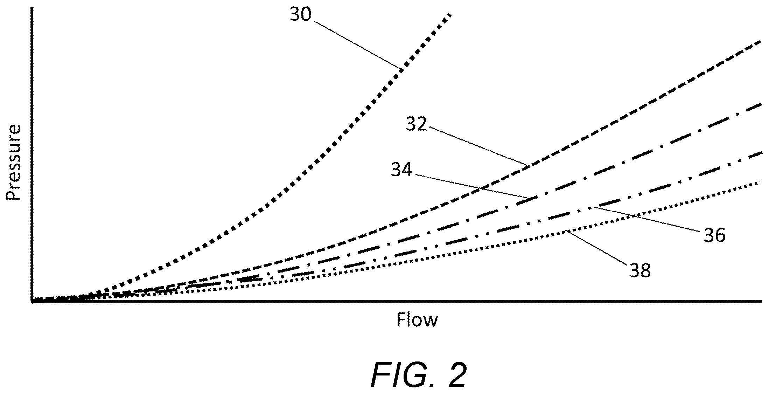

As an example, Figure 2 illustrates the resistance to flow for various different sized 34, 36, 38, 30, 32 non-sealing nasal cannulas, expressed in terms of the pressure drop across the cannula for different flow rates. It can be seen that the different sized cannulas each have different resistance to flow characteristics.

The cannulas with higher resistance to flow are represented by the plots that indicate relatively high pressure drops at the lower flow rates, such as plot 30 and plot 32. Appliances with lower resistance to flow are represented by the plots that indicate relatively low pressure drops such as plot 34, plot 36 and plot 38.

### Connector assembly

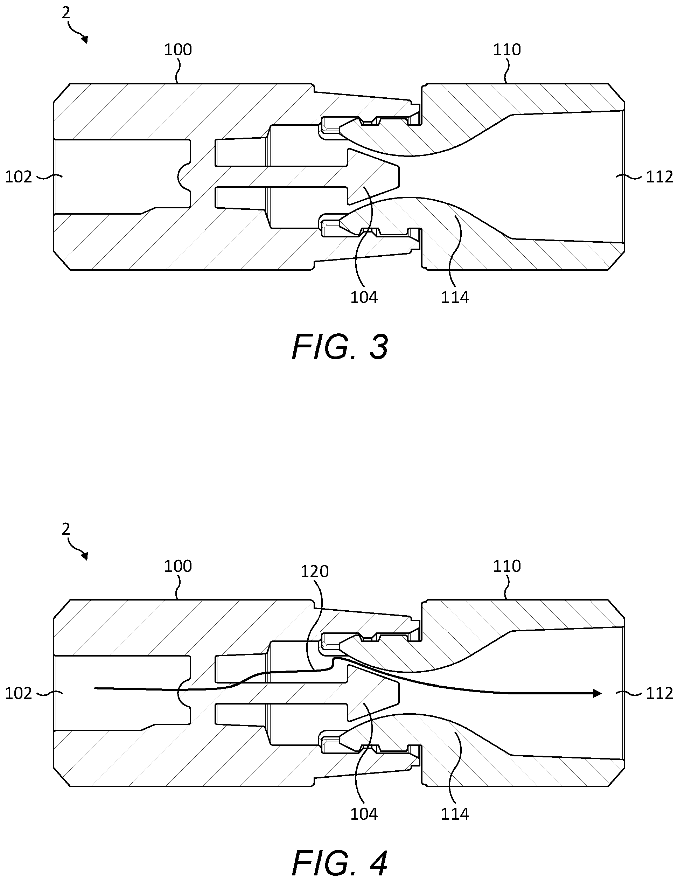

Referring to Figure 3 and Figure 4, the connector assembly 2 comprises a first connector 100 and a second connector 110. The first and second connectors are releasably engageable to each other to connect or release the patient interface and any associated conduits and/or filters to/from the rest of the respiratory circuit. When connected, the first and second connectors define a gas flow path therethrough.

The connector assembly 2 when assembled forms a flow modifying arrangement. The flow modifying arrangement comprises a first part 104 provided on the first connector 100, and a second part 114 provided on the second connector 110. The flow modifying arrangement is configured to change a property of a gas flow, such as pressure, along the gas flow path when the first and second connectors 100, 110 are connected.

The first and second connectors, 100, 110 are arranged co-axially, with the first connector 100 typically upstream of the second connector 110. When the first and second connectors are connected, the first part of the flow modifying arrangement is positioned proximate to the second part.

The first and second parts of the flow modifying arrangement are disposed in the gas flow path and arranged to be positioned relative to each other in a predetermined manner when the first and second connectors 100, 110 are engaged. When the connectors 100, 110 are engaged, the first and second parts of the flow modifying arrangement together define a high resistance flow path 120 through the flow modifying arrangement. The first and second parts of the flow modifying arrangement form a dominant flow restriction in a respiratory system or respiratory conduit. The dominant flow restriction may decouple the upstream and downstream portions of the connector assembly, by way of the first and second connectors of the connector assembly being disconnected.

The first part 104 of the flow modifying arrangement is spaced from the second part 114. Depending on the nature of the first part and the second part, the spacing may be axial, or the parts may overlap in the axial direction and the spacing may be in a generally radial direction, such as for the embodiment shown in Figure 4. The size of the spacing is selected to provide a pre-determined change to a property of gas flow along the gas path 120.

The flow path 120 through the flow modifying arrangement has a higher resistance to flow than portions of the flow path immediately upstream and downstream of the flow modifying arrangement. That is, the flow modifying arrangement may induce a significant increase in the velocity of the fluid flow therethrough, may cause a significant pressure drop, may make the flow at the flow modifying arrangement at least partially turbulent if flow is laminar, and/or may increase the turbulence of the flow at the flow modifying arrangement if flow is already turbulent. The flow modifying arrangement is configured to result in a significant change in one or more properties of the fluid flow along the gas the flow path 120 from a point immediately upstream of the flow modifying arrangement compared to a point immediately downstream of the flow modifying arrangement.

In some embodiments, the first and second parts 100, 110 of the flow modifying arrangement cause an increased resistance to flow by being positioned relative to each other in a manner that defines a tortuous flow path through the flow modifying arrangement, such as, for example, the flow path 120 shown in Figure 4. In other embodiments, the first and second parts of the flow modifying arrangement may cause an increase in resistance to flow by defining a flow path with a significantly reduced or narrowed cross sectional area compared to the flow path upstream and downstream of the connection assembly.

The size and/or shape of the fluid flow path through the flow modifying arrangement is selected to provide a pre-determined change to a property of gas flow across the flow modifying arrangement. The embodiments of the first and/or second parts of the flow modifying arrangement described could in some forms be formed from projections from the wall of the connector, or be formed integrally with the wall of the connector. In some forms, the flow modifying geometries described such as bars, orifice plates etc. can comprise additional surface roughness or texture in order to alter the pressure drop and thereby the RTF of the flow modifying arrangement. The first and/or second parts of the flow modifying arrangement may allow for tolerance in the alignment of the two parts of the flow modifying arrangement in that they allow the two parts of the flow modifying arrangement to move relative to each other without undesirably impacting the RTF of the flow modifying arrangement. That is, the first and/or second parts of the flow modifying arrangement may be configured such that the resistance to flow through the connector assembly is not overly sensitive to relative movement of the two parts within a selected tolerance.

The first part 104 of the flow modifying arrangement may be a constriction or an obstruction. A constriction acts to provide a narrowing of the fluid flow path, reducing the cross-sectional area of the flow path at the constriction. The narrowing may be gradual or sudden. The constriction may be integral with the respective connector, for example the constriction may be formed by an increase in the wall thickness of the connector.

A suitable obstruction is a partial obstruction that blocks a portion of the gas flow path but allows gases flow around the obstruction, along the gas flow path. The obstruction is disposed in the gas flow path. It may be integral with the wall of the respective connector, or it may be a separate member that is fixed to the wall of the connector.

Depending on the nature of the first part 104, the second part 114 of the flow modifying arrangement may be a constriction or an obstruction shaped and positioned to interact with the first part 104. In the embodiment of Figures 3 and 4, the first part 104 of the flow modifying arrangement comprises and obstruction and the second part 114 of the flow modifying arrangement comprises a constriction, however, other arrangements are envisaged and discussed below.

Typically, the first connector 100 and thereby the first part 104 of the flow modifying arrangement is provided on the multi-use part of a system, for example coupled to an inspiratory conduit or filter for attaching to single-use componentry. It may be integral with the conduit. In such an arrangement, the second connector 110 and thereby the second part 114 of the flow modifying arrangement is provided on the single-use part of the system, for example on the patient interface, filter, or a conduit leading to the patient interface. Conversely the first part 104 of the flow modifying arrangement may be provided on the patient interface, with the second connector 110 and second part 114 of the flow modifying arrangement provided on the inspiratory conduit. In such an embodiment, the inspiratory conduit and associated second connector may be switched when moving between respiratory systems. In alternative forms, the first connector 100 and thereby the first part 104 of the flow modifying arrangement is provided elsewhere in the respiratory circuit, such as at or near the humidifier or humidification chamber. Similarly, the second connector 110 and thereby the second part 114 of the flow modifying arrangement may be alternatively provided elsewhere in the respiratory circuit, for example on the conduit which connects to the humidifier or humidification chamber.

For a given system the first part of the flow modifying arrangement may be predetermined, however, the nature of the second part of the flow modifying arrangement on the single-use part of the system may vary for different sizes patient interface to ensure the resistance to flow of the system as a whole is generally the same or remains with in an acceptable range for the system regardless of the interface that is connected.

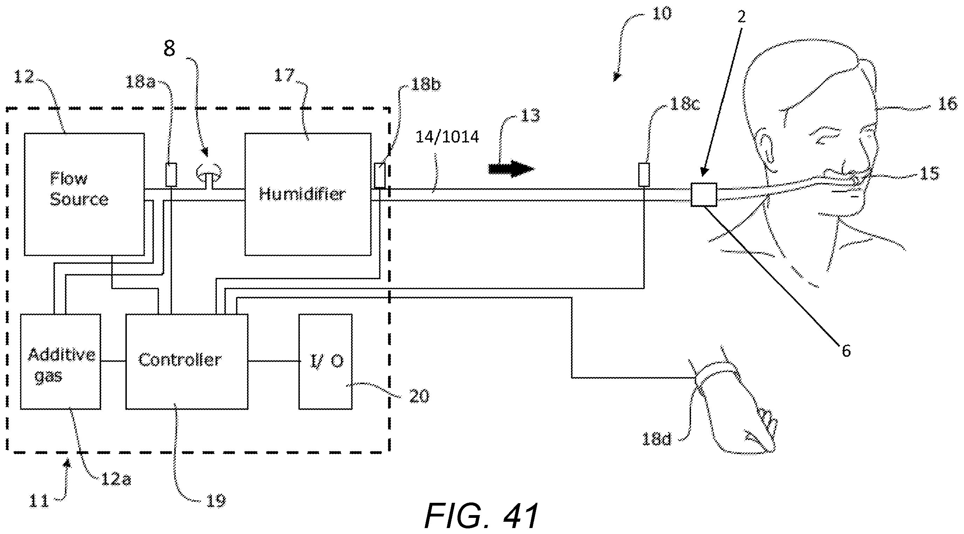

The first and second parts of the flow modifying arrangement 104, 114 provided in each connector 100, 110 are shaped such that each part of the flow modifying arrangement does not substantially alter a property of a gas flow across that connector, when it is not connected to the other connector and is instead coupled for example to a regular conduit or other connector without a flow modifying arrangement part or portion. Figure 41 shows a respiratory system similar to system 10 as shown in Figure 1, with only a second connector 6 located within the respiratory circuit. The second connector 6 comprises the second part of the flow modifying arrangement. This configuration may be beneficial in the respiratory system shown where the flow source and respiratory circuit may have different driving pressure requirements or other flow delivery requirements which can be impacted if a property of a gas flow is altered. The first or second part of the flow modifying arrangement 104, 114 may cause some change in flow across the connector even when it is not connected to the other connector 100, 110 and the other of the flow modifying arrangement part 104, 114, however, this change in resistance to flow is minimal, insignificant or negligible compared to the change in resistance to flow caused by the assembled flow modifying arrangement. Ensuring the change in resistance to flow is minimal, insignificant or negligible across a connector when it is connected to a component other than the other connector ensures that when any component the connector is associated with, such as the patient interface or conduit, is used in another respiratory system (which may have different pressure requirements), the introduction of the connector into that system does not substantially impact the driving pressure requirements of the system.

That is, the first part of the flow modifying arrangement 104 is shaped and positioned such that it causes only a minimal, insignificant or negligible change to the selected property of a gas flow across the first connector 100, when the first connector is separate from the second connector. Similarly the second part of the flow modifying arrangement 114 is shaped and positioned such that it causes only a minimal, insignificant or negligible change to the selected property of a gas flow across the second connector 110, when the second connector is separate from the first connector.

For example, in one embodiment system has a flow rate of 70 L/min, the first connector 100 individually causes a first pressure drop across the connector, and the second connector 110 individually causes a second pressure drop across the connector, whereas in combination the connection assembly, including the first and second parts of the flow modifying arrangement 104, 114 causes a third pressure drop that is higher than the first and second pressure drops. In one exemplary embodiment system with a flow rate of 70 L/min and including a pressure relief valve, the respiratory system has a resistance to flow of between about 30 and about 50 cmH2O at 70 L/min.

The first connector 100 is releasably engageable with the second connector 110 to connect and disconnect the two connectors from each other. The first connector 100 may comprise a first engagement feature for engaging with the second connector 110 or a complementary engagement feature on the second connector. The engagement features may for example comprise a snap-fit connection, a threaded connection, a catch, or be of any suitable type that will be known to those skilled in the art. Alternatively, the first and second connectors may attach by way of a press-fit. In some embodiments, an intermediate connecting member may be provided for coupling the first connector to the second member. When connected, the first and second connectors define a gas flow path therethrough.

The first and second connectors 100, 110 may additionally comprise alignment features, to orientate the first part of the flow modifying arrangement 110 relative to the second part of the flow modifying arrangement 114. Alignment features may particularly be included where the angle of alignment or angular alignment of the first part of the flow modifying arrangement relative to the second part of the flow modifying arrangement is important to ensure correct assembly or correct function of the flow modifying arrangement.

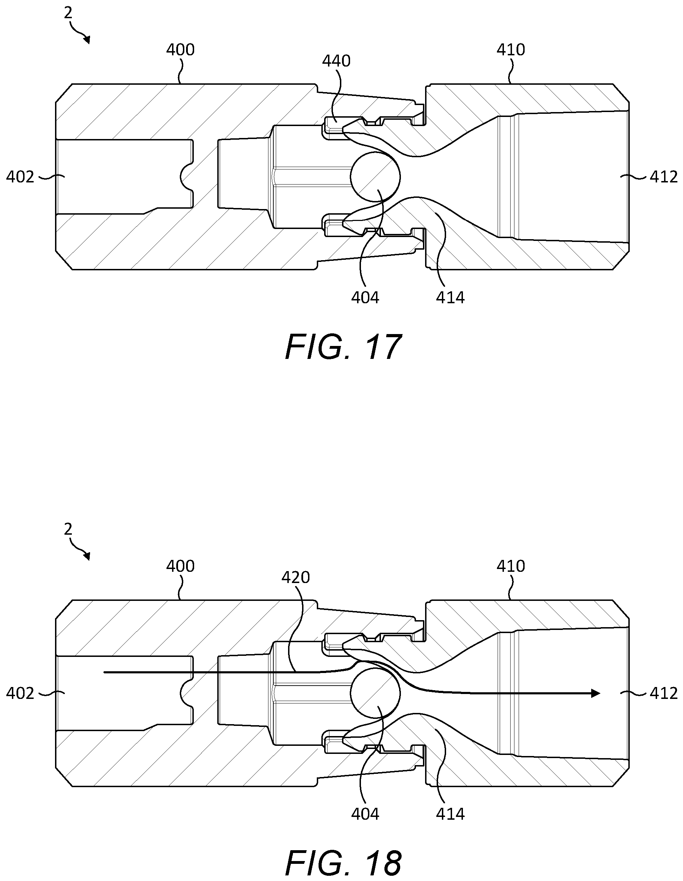

The alignment features may be internal or external. The alignment features may comprise a guide, such as a channel 440 (Figure 17), or a stop, or may include visual markings for a user to align when assembling the circuit. The design of the interaction between the two connectors 100, 110 will preferably be such that there is some tolerance on the location of the first and second parts of the flow modifying arrangement (how close they need to be to one another to sufficiently change the resistance to flow). This may allow some movement in the connection between the connectors, which effects the first and second parts of the flow modifying arrangement, and for example the tube and cannula.

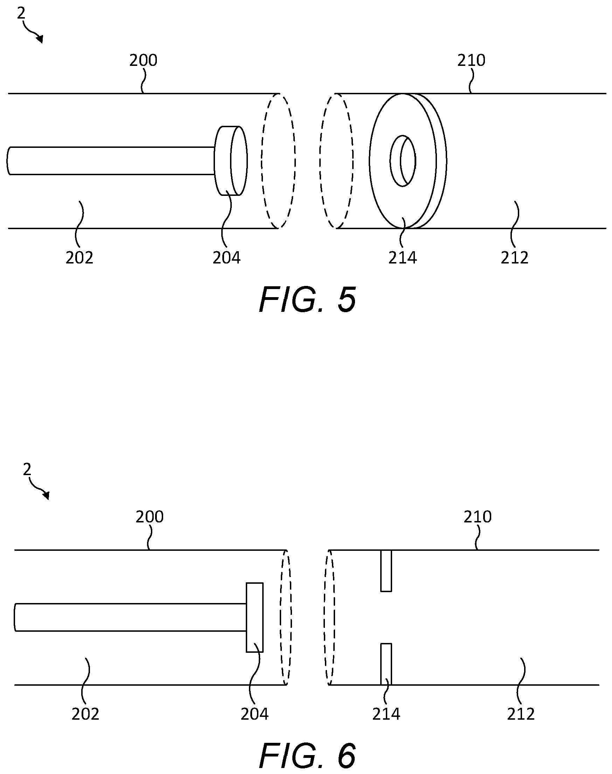

Referring to Figures 5 to 12, a simplified schematic of the parts of one embodiment of a connector assembly is shown. In this embodiment, the connector comprises a first connector 200 and a second connector 210. The first and second connectors are releasably engageable to each other to connect or release the patient interface to/from the rest of the respiratory circuit.

The connector assembly 2 when assembled forms a flow modifying arrangement. The flow modifying arrangement comprises a first part 204 in the form of an obstruction provided on the first connector 200, and a second part 214 in the form of a constriction provided on the second connector 210. The flow modifying arrangement is configured to change a property of a gas flow, such as pressure, along the gas flow path when the first and second connectors 200, 210 are connected.

The first and second parts of the flow modifying arrangement 204, 214 are disposed in the gas flow path and arranged to be positioned relative to each other in a predetermined manner when the first and second connectors engage. The first part of the flow modifying arrangement 204 is disposed in the gas flow path 202 of the first connector 200, and the second part of the flow modifying arrangement 214 is disposed in the gas flow path 212 of the second connector 210.

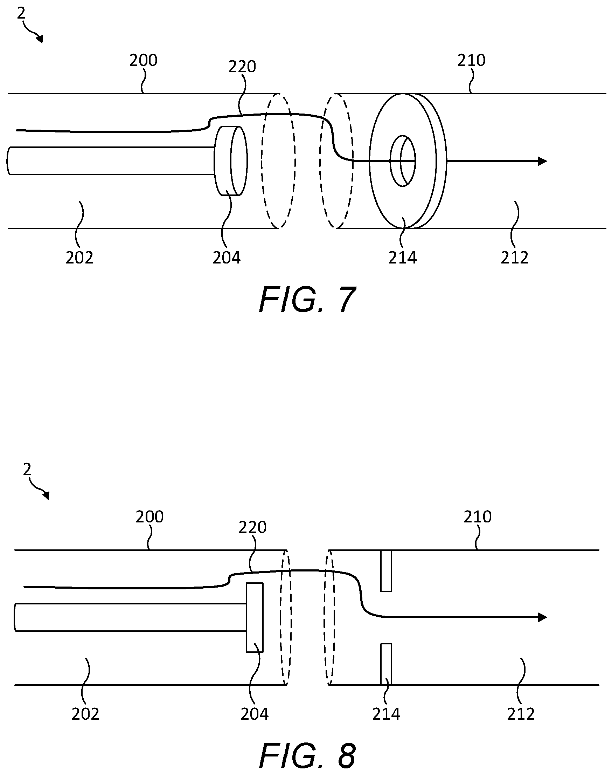

As shown in Figure 7 and Figure 8, the flow path defined by the flow modifying arrangement and represented by arrow 220 through the flow modifying arrangement has a higher resistance to flow than portions of the flow path immediately upstream and downstream of the flow modifying arrangement. When the connectors 200, 210 are engaged, flow 220 through the connection assembly is forced to take a narrowed and winding, torturous path through the flow modifying arrangement, thereby increasing the resistance to flow and the pressure drop over the connection assembly. The flow modifying arrangement causes an increase in the velocity of the fluid flow at the flow path through the flow modifying arrangement.

Each connector 200, 210 and the respective part of the flow modifying arrangement 204, 214 is shaped such that it does not substantially alter a property of a gas flow across that connector, when it is not connected to the other connector and is instead coupled for example to a regular conduit or other connector without a flow modifying arrangement part or portion.

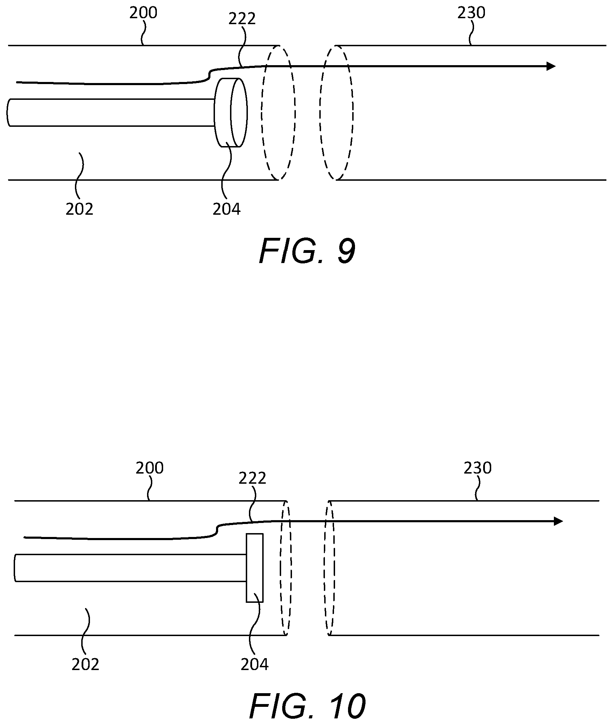

Figures 9 to 12 illustrate the flow path 222, 224 when the connectors are connected to regular conduits without flow modifying portions. As previously noted, the first or second part of the flow modifying arrangement 204, 214 may cause some change in flow across the connector even when it is not connected to the other connector 200, 210 and the other of the flow modifying arrangement part 204, 214, however, this change in resistance to flow is minimal, insignificant or negligible compared to the change in resistance to flow caused by the assembled flow modifying arrangement.

Figures 9 and 10 illustrate the first connector 200 as previously described connected to a further connector 230 that does not comprise a flow modifying arrangement. In this exemplary arrangement, the first part of the flow modifying arrangement 204 may cause some deviation in flow path 222 across the connector 200 compared to a system where the flow obstruction 204 is absent. However, this change in the flow path 222, and the resulting resistance to flow it causes is minimal, insignificant or negligible compared to the change in resistance to flow caused by the assembled flow modifying arrangement of the connector assembly shown in Figures 5 to 8.

That is, the first part of the flow modifying arrangement 204 is shaped and positioned such that it causes only a minimal, insignificant or negligible change to the selected property of a gas flow across the first connector 200, when the first connector is separate from the second connector.

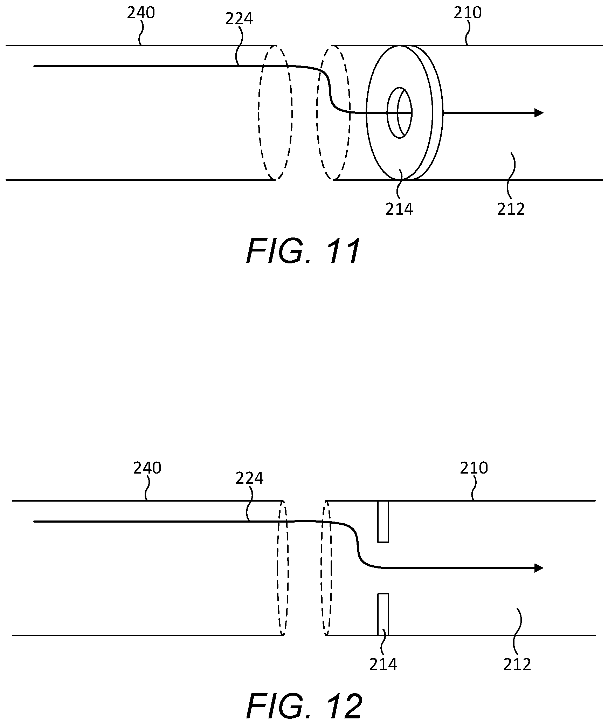

Similarly, as shown in Figure 11 and Figure 12, where the second connector 210 as previously described is connected to a fourth connector 240 that does not comprise a flow modifying arrangement, the change in the flow path 224, and the resulting resistance to flow it causes is minimal, insignificant or negligible compared to the change in resistance to flow caused by the assembled flow modifying arrangement of the connector assembly shown in Figures 5 to 8.

That is, the second part of the flow modifying arrangement 214 is shaped and positioned such that it causes only a minimal, insignificant or negligible change to the selected property of a gas flow across the second connector 200, when second connector 210 is separate from the first connector 240.

More specific embodiments of the connection assembly are described below in relation to the drawings. These embodiments are not intended to be limiting and variations are anticipated.

### Blocking 'plate' embodiments

Figures 3, 4, and 13 to 16 illustrate embodiments of the connector assembly 2, where the first part of the flow modifying arrangement 304 comprises a flow obstruction in the form of a blocking member positioned axially and disposed in the gas flow path 302 of the first connector 300, coupled with a constriction forming the second part of the flow modifying arrangement 314 disposed in the gas flow path 312 of the second connector 310.

In these embodiments, the blocking member 304 comprises a longitudinal axis that is substantially parallel to a longitudinal axis of the first connector 300. The blocking member 304 is positioned or disposed in the gas flow path 302 of the first connector, with the longitudinal axis of the blocking member generally co-linear with the longitudinal axis of the first connector 300.

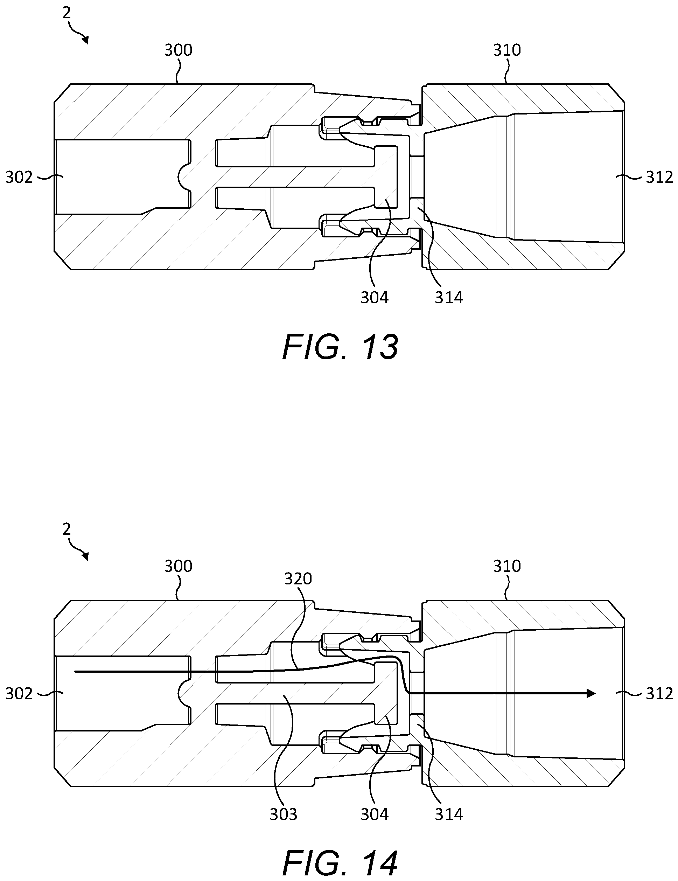

As shown in these embodiments, the blocking member 304 comprises an elongate positioning portion 303 for positioning the blocking member 304 in the flow and attaching the blocking member to the wall of the connector 300 at a point spaced from the end of the connector. The positioning portion 303 in the embodiment shown is positioned co-axially with the first connector and preferably has a minimal, insignificant or negligible impact on flow through the connector. The positioning portion attaches to the blocking member 304. In the embodiment shown of Figures 13-16, the blocking member comprises a substantially cylindrical plate 304. The cylindrical plate 304 has a greater radial diameter than that of the elongate positioning portion 303.

In other embodiments the blocking member may have other shapes. For example, at least one portion of the blocking member may have a variable radial diameter. The portion(s) of variable radial diameter may be such that at least a portion of the edges of the longitudinal section of the blocking plate are linear or straight. In these embodiments, the variable radial diameter of the blocking plate may mean the blocking member takes one or more of the following annular forms: cylindrical, frustoconical, cuboidal, pyramidal, prismatic, conical.

In some embodiments, the blocking member may be otherwise shaped. One or more of the portion(s) of variable radial diameter are such that at least one of the edges of a portion of longitudinal section of the blocking member may be non-linear or curved. The blocking member may be substantially spherical, hemispherical, hyperboloid, lens, ellipsoid, lemon, or otherwise shaped.

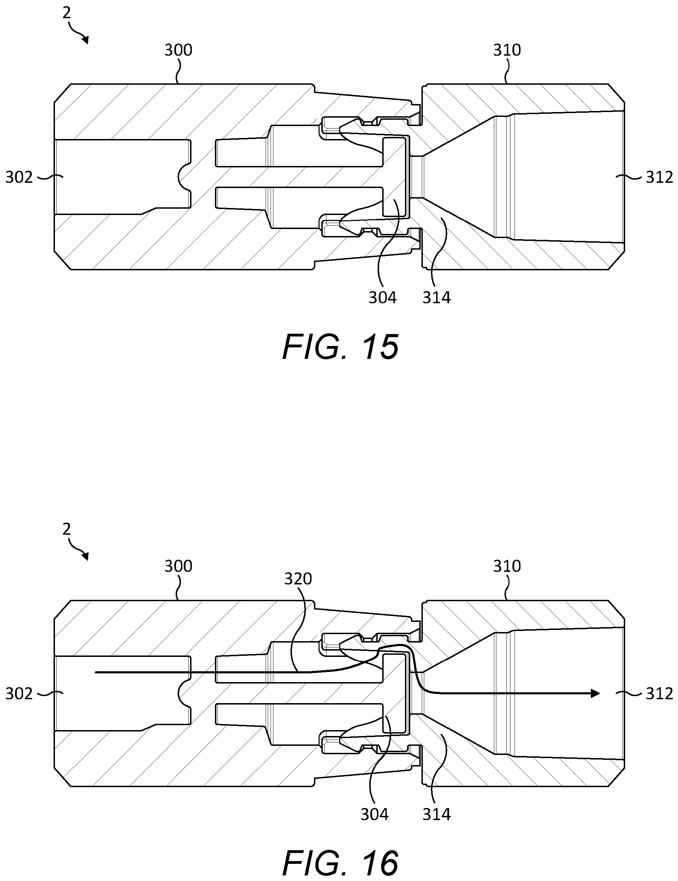

As shown by the example gas flow 320 through the connector assembly 2 (Figure 16), the blocking member 304 acts to reduce the cross-sectional area of the gases flow path 302 of the first connector 300, and to force flow towards the annular perimeter of the connector. As shown, the blocking member 304 is arranged proximate to the constriction to define a tortuous gas flow path 320 through the connector assembly.

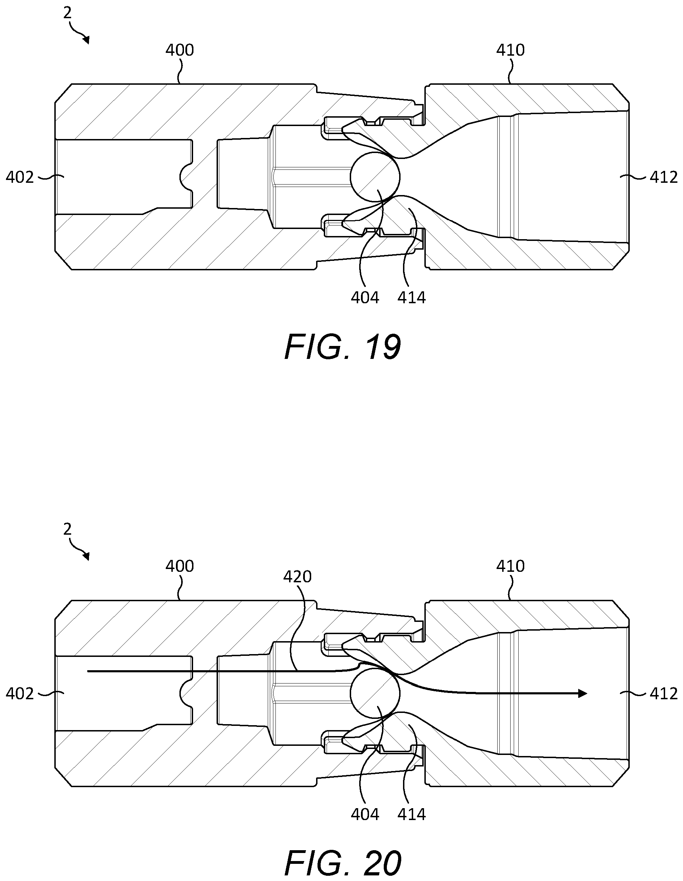

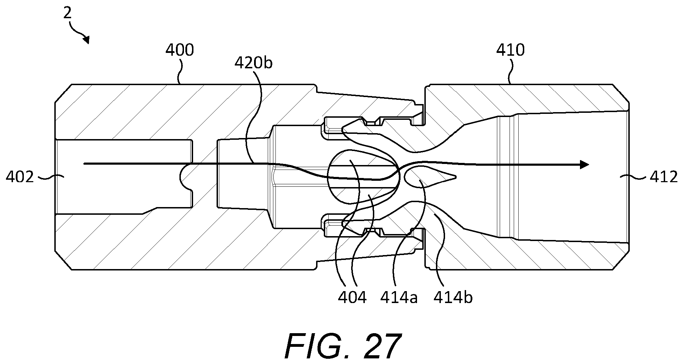

The constriction forming the second part of the flow modifying arrangement 314 is disposed in the gas flow path 312 of the second connector 310. The constriction defines a portion of the second connector 310 having a reduced internal diameter along the gas flow path 312. The constriction in may be in the form of an orifice, as shown in Figures 13 and 14, or in the form of a venturi (Figures 17 to 27 discussed below), or a combination thereof (Figures 15 and 16).

Referring to Figures 13 and 14, an orifice plate 314 is created by an inwardly extending annular protrusion from the wall of the second connector 310. The protrusion defines a portion of the second connector 310 with a reduced internal diameter, such that an orifice is formed. The protrusion may be integral with the wall of the connector such that in effect, the orifice is formed from a thickened portion of a wall of the connector. In other embodiments, the orifice may be formed by an orifice plate disposed in the flow path of the second connector 310.

In the embodiment of Figures 15 and 16, the second part of the flow modifying arrangement is a constriction comprising an orifice with a tapered portion on the downstream side of the orifice . The orifice defines a portion of the second connector 310 having a reduced internal diameter along the gas flow path 312. The tapered portion defines a length of the orifice with a variable internal diameter along at least a portion of the longitudinal axis of the secondary connector 310.

In embodiments having a tapered portion on the downstream side of the orifice forming the second part of the flow modifying arrangement, the tapered portion may aid in the recovery of a pressure drop as compared to embodiments having an orifice. This, through smoothing i.e. the gradual transition/taper, of the internal wall of the cannula end after the orifice, reduces pressure loss after the orifice, and has improved pressure recovery when compared to embodiments featuring only an orifice, such as that shown in Figures 13 and 14.

In other embodiments, the second, first, or both parts of the flow modifying arrangement may comprise a venturi, the profile of the venturi may be linear or curved or a combination. The venturi may be annular or may also extend partway around the perimeter of the connector, such that it is substantially planar and is made up of the two sides of the venturi. The venturi may be asymmetrical about a transverse plane at a throat of the venturi, and the throat is arranged proximate the blocking member of the first connector. In the embodiment shown in Figures 3 and 4, the second part of the flow modifying arrangement 114 is an annular venturi.

Embodiments having a venturi, when compared to embodiments having an orifice or bar, may have features such as more gradual transitions, converging or diverging portions, and lead in or lead out angles (for example, a 12-degree angle). Any one or more of these features may act to reduce flow separation through the venturi. Flow separation can typically cause pressure loss, and so embodiments having a venturi, when compared to embodiments having an orifice or bar may provide a substantially improved pressure recovery.

As previously discussed, the first part of the flow modifying arrangement, in the presently described embodiments being in the form of a blocking member, is arranged proximate to the second part of the flow modifying arrangement to define a tortuous gas flow path through the flow modifying arrangement. It will be appreciated that the size of the blocking member and/or the size of the orifice or venturi is selected to provide a desired resistance to flow through the flow modifying arrangement. As such, the size of the orifice or the size of the opening through the constriction of the second part of the flow modifying arrangement in the flow path will be different for different downstream components. Similarly, the size of the gap around the blocking plate can be altered by increasing or decreasing the size of the plate. In embodiments having a venturi, characteristics of the venturi can be varied to control the size of the constriction and/or the pressure recovery of the venturi. These characteristics may include one or more of the following: the length of the venturi or the curved portion of the venturi, the length of the converging and/or diverging portions of the venturi, the radius of curvature about the maximum constriction of the venturi, the rate of transition of the converging and/or diverging portions of the venturi.

Together, the first and second parts of the flow modifying arrangement create a narrowed and non-linear, tortuous flow path 320 around the blocking member 304 and through the orifice or venturi 314, which in turn increases the resistance to flow in the gases flow path.

The distance between the blocking plate and the orifice or venturi can be varied to alter the restriction and thus the characteristics of the tortuous flow path and the subsequent resistance to flow provided by the flow modifying arrangement.

The size and shape of the orifice and/or venturi is intended to be varied between different cannulas, the cannulas having different sizes. By varying the orifice and/or venturi provided in the wall protrusion, for example by having the orifice and/or venturi larger in smaller sizes of cannula, and the orifice and/or venturi smaller in larger sizes of cannula, the flow modifying arrangement is intended to provide the same or substantially similar resistance to flow, when the orifice and/or venturi is combined with the blocking member or first part of the flow modifying arrangement. A smaller orifice or venturi provides a narrowed constriction for the gases in the flow path, and as such increases the resistance to flow of the flow modifying arrangement more than a larger orifice or venturi. This allows the substantial matching of the resistance to flow of a larger sized cannula, which without the flow modifying arrangement would have a smaller resistance to flow, to a smaller sized cannula, which without the flow modifying arrangement would have a larger resistance to flow. The initial differences between cannulas which result in a different resistance to flow could be differences in diameter and/or length of the prongs, and/or size of the cannula body or manifold.

### Cross bar and venturi embodiments

Figures 17 to 27 illustrate embodiments of the connector assembly 2, where the first part of the flow modifying arrangement 404 comprises an alternative form flow obstruction in the form of one or more 'bars' positioned axially and disposed in the gas flow path 402 of the first connector 400, coupled with the second part of the flow modifying arrangement 414 comprising a constriction in the form of a venturi disposed in the gas flow path 412 of the second connector 410.

In these embodiments, the, or each, bar 404 extends across at least a portion of the flow path 402 of the first connector 400. In some embodiments, the, or each, bar 404 is a crossbar arranged substantially transverse to the flow path. In such embodiments, the crossbar comprises a longitudinal axis that is substantially transverse to a longitudinal axis of the first connector 400.

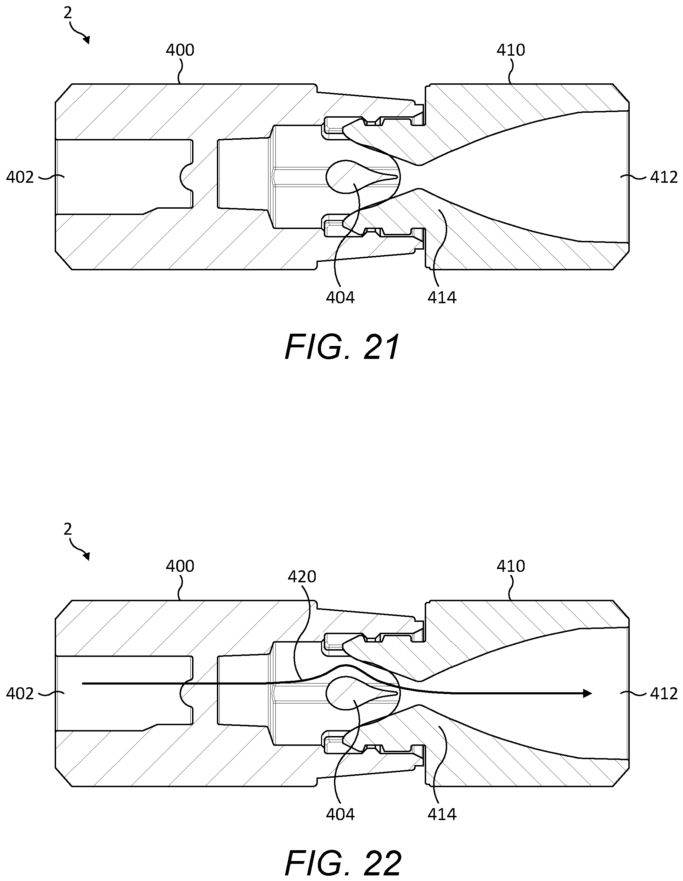

The bar or bars 404 have a cross-sectional profile that can take a number of different forms depending on the desired effect on the property or properties of the gas flow along the gas flow path. As shown in Figures 17 to 20, in its most basic form, the bar 404 may be cylindrical with a substantially circular cross-sectional profile. Figures 21 and 22, illustrate an embodiment in which the bar 404 has a substantially tear drop shaped cross-sectional shape.

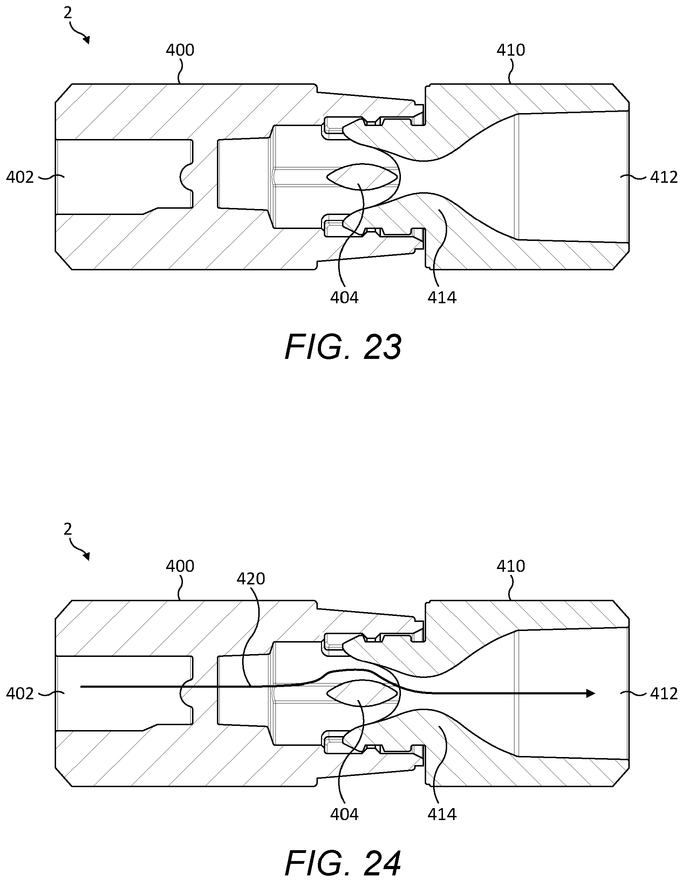

In some embodiments, the bar is an aerofoil, or may have one or more curved surfaces. In such embodiments, the curved surfaces of the bar may reduce flow separation. Flow separation can cause pressure loss, and so embodiments having one or more curved surfaces may provide improved pressure recovery. As further examples, Figures 23 and 24 show a bar 404 with a substantially biconvex cross-sectional shape. Other shapes are envisaged and within the ambit of this application.

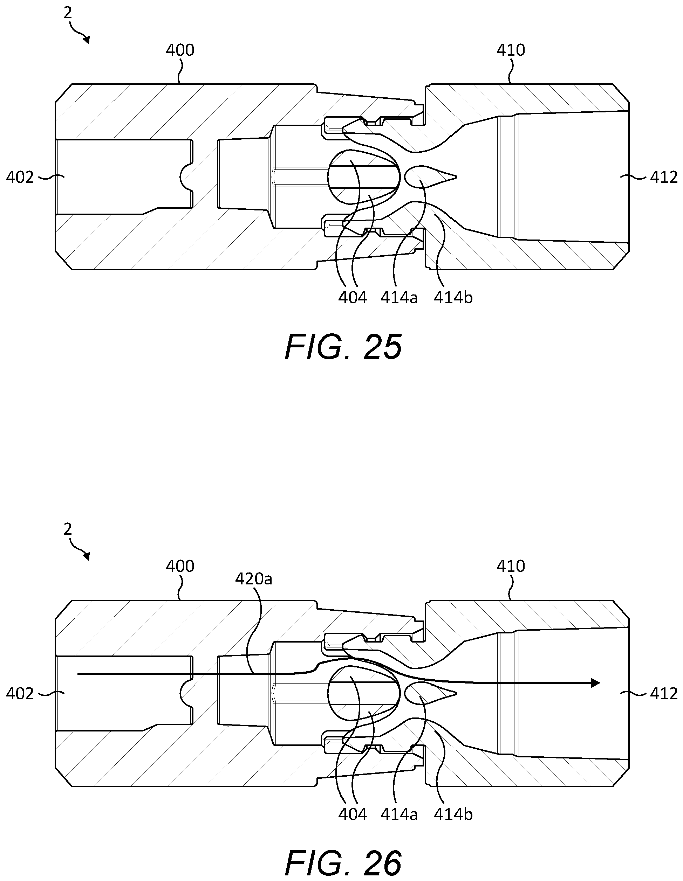

Where multiple bars are included, these may be identically formed, or their shape may vary. Where the shapes vary or are differently orientated, they may be complementary as shown in the embodiment of Figures 25 to 27. In that embodiment two bars 404 are provided, each having a substantially semi-circular cross-sectional shape. In other embodiments, the, or each, bar may have a substantially quadrilateral, cross-shaped, triangular, or elliptical, cross-sectional shape.

As shown by the example gas flow 420 through the connector assembly 2, the bar or bars 404 act as an obstruction and reduce the cross-sectional area of the gases flow path 402 of the first connector 400. Flow is forced towards the annular perimeter of the connector. As shown, the bar or bars 404 are configured to be arranged proximate to the second part of the flow modifying arrangement to, in combination, define a tortuous gas flow path 420 through the connector assembly.

In these embodiments or any other, the, or each, bar extends across at least a portion of the flow path 402 of the first connector 400 from wall to wall, substantially transverse to a longitudinal axis of the first connector 400. In some embodiments, the, or each, bar extends all of the way across the flow path of the first connector from wall to wall. In other embodiments, the, or each, bar may extend a desired length, for example between 90% to 60% of the way from a single wall, or preferably between 80% to 70% of the way from a single wall.

The constriction forming the second part of the flow modifying arrangement 414 is similarly disposed in the gas flow path 412 of the second connector 410. The constriction defines a portion of the second connector 410 having a reduced internal diameter along the gas flow path 412. The constriction in may be in the form of an orifice, as previously described, and as shown in Figures 13 and 14, in the form of an annular venturi, shown for example in Figures 15 and 16, or more preferably in the form of a planar venturi, as will be described below and with reference to Figures 17 to 27.

In the embodiments shown in Figures 17 to 27, the second part of the flow modifying arrangement comprises a planar venturi 414. The planar venturi 414 is one which protrudes into the flow path of the second connector 410 to define a flow path through the constriction that is substantially planar, rather than protruding annularly around the circumference of the second connector to define a circular opening through the constriction.

The planar venturi defines a portion of the second connector 410 having a reduced internal diameter along the gas flow path 412, and has a variable internal diameter along at least a portion of the longitudinal axis of the secondary connector 410. As shown in Figures 17 to 20, and Figures 23 and 24, in some embodiments, the profile of the planar venturi may be curved. In other embodiments, as shown in Figures 21 and 22, the profile of the planar venturi may include one or more linear portions. In other embodiments, the planar venturi is asymmetrical about a transverse plane at a throat of the venturi, and the throat is arranged proximate the bar or bars of the first connector.

In an exemplary embodiment, the first or second part of the flow modifying arrangement may comprise a venturi. Embodiments having a venturi may have features such as more gradual transitions, converging or diverging portions, and lead in or lead out angles (for example, a 12-degree angle). Any one or more of these features may act to reduce flow separation through the venturi. Flow separation can typically cause pressure loss, and so embodiments having a venturi, when compared to embodiments having an orifice or bar may provide a substantially improved pressure recovery.

Figures 25 to 27 show another example embodiment of the second part of the flow modifying arrangement 414, with a modification to the restriction in the second connector 410. In this embodiment, the second part of the flow modifying arrangement comprises a curved, planar venturi 414b, and a bar 414a. In this example embodiment, the bar has a substantially tear dropped shaped cross section, but it could also be any of the previously mentioned shapes. The first part of the flow modifying arrangement comprises two cross bars 404 having substantially semi-circle shaped cross sections. The cross bars 404 that are positioned to be above and below the tear drop shaped cross bar 414a of the second part of the flow modifying arrangement. When the connectors are connected, and the flow modifying arrangement is formed, two or more tortuous flow paths 420a, 420b are created by the proximity of the first and second parts of the flow modifying arrangement, the tortuous flow paths acting to alter the resistance to flow within the gas flow path, as previously described.

In some embodiments, more than one bar may be used in the first part of the flow modifying arrangement in the first connector. In some embodiments, one or more of the bars may have different cross sectional shapes, or may extend through different portions across the flow path. Additionally, or alternatively, it will be appreciated that there may be more than one component comprising the second part of the flow modifying arrangement in the second connector. The one or more components of the second part of the flow modifying arrangement may not necessarily be the same type, for example the components of the second part of the flow modifying arrangement could comprise a cross bar and a curved venturi, or any other combination of the blocking member, orifice, annular venturi described in relation to any of the previous embodiments.

### Other embodiments

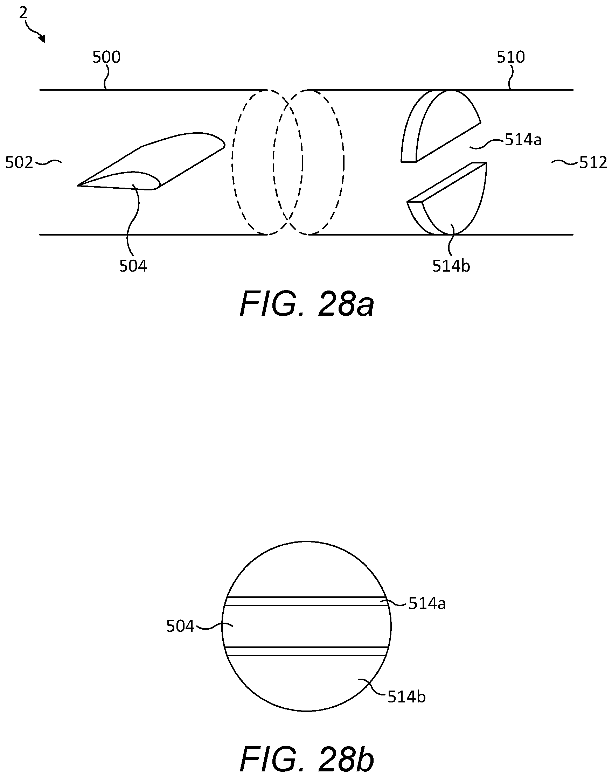

Referring now to Figures 28a and 28b, another embodiment of the combination of the components of the first and second parts of the flow modifying arrangement are shown. This embodiment comprises a cross bar 504 as the first part of the flow modifying arrangement, displaced within the flow path 502 of the first connector 500, and an orifice 514a as the second part of the flow modifying arrangement, the orifice 514a created from two semi-circle protrusions 514b displaced within the flow path 512 of the second connector. It will be appreciated that the bar 504 may take on any of the cross-sectional shapes previously mentioned. Figure 28b shows a front on cross-sectional view of the connector assembly 2 from downstream of the protrusions 514b, showing the interaction between the geometries of the bar 504 and the orifice 514a created by the two protrusions 514b. The interaction of the orifice 514a with the bar 504 creates the tortuous flow path that changes the resistance to flow in the flow path between the connector assembly as desired.

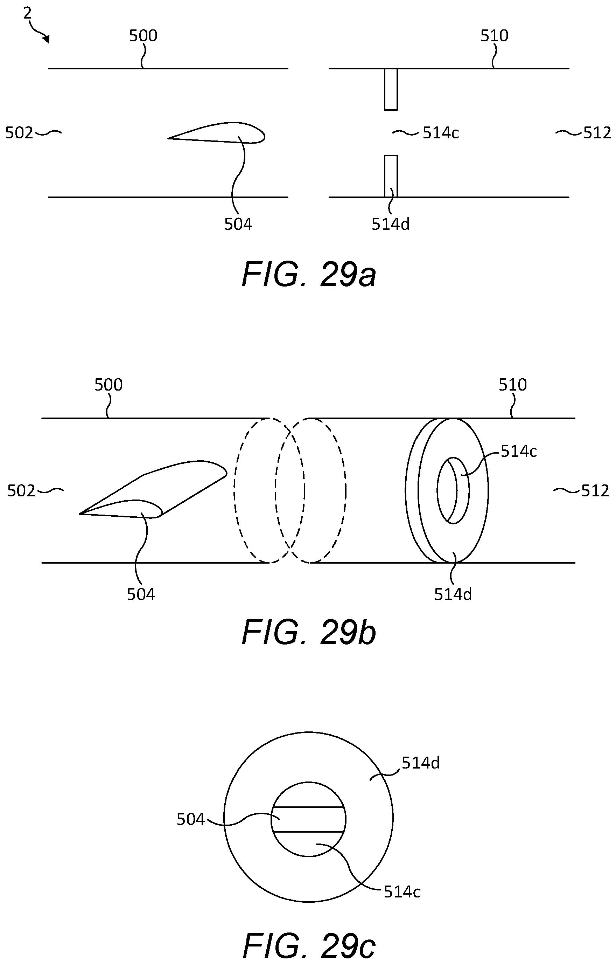

A similar embodiment is shown in Figures 29a, 29b, and 29c, comprising a cross bar 504 as the first part of the flow modifying arrangement, displaced within the flow path 502 of the first connector 500, and a circular orifice 514c as the second part of the flow modifying arrangement, the circular orifice 514c created from an annular protrusion or orifice plate 514d displaced within the flow path 512 of the second connector.

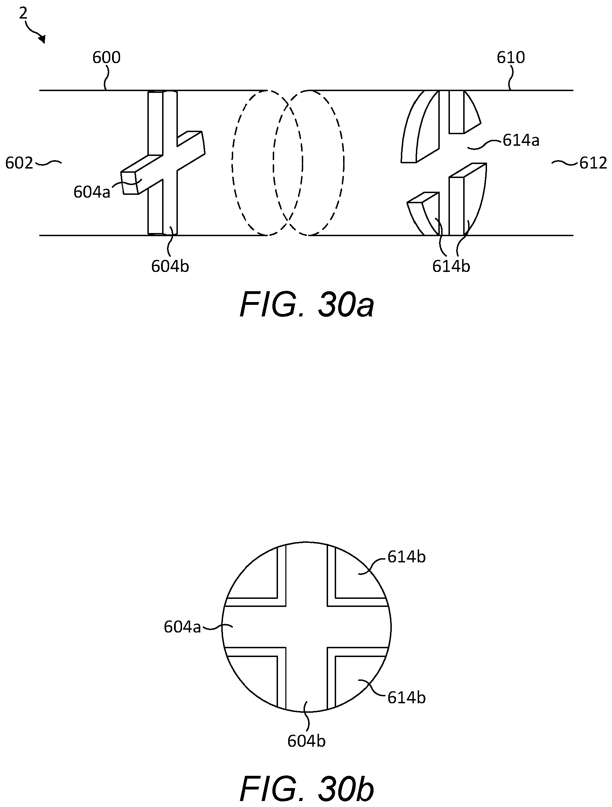

Another possible embodiment is shown in Figures 30a and 30b, and comprises two cross bars 604a, 604b that intersect and form a cross shaped bar as the first part of the flow modifying arrangement, displaced within the flow path 602 of the first connector 600. The second part of the flow modifying arrangement comprises an orifice 614a. The orifice 614a is has an enlarged cross sectional shape to the cross section of the cross shaped bar, or, if the two cross bars 604a, 604b are separate components that formed the cross shaped bar, the orifice has an enlarged cross sectional shape to the cross section of the interaction of these two cross bars 604a, 604b. As shown, the orifice may be created from four quarter circle protrusions 614b displaced within the flow path 612 of the second connector 610, to create a plus sized orifice. The four quarter circle protrusions 614b in this embodiment line up with the cross shaped cross bar when the first and second connectors are connected together.

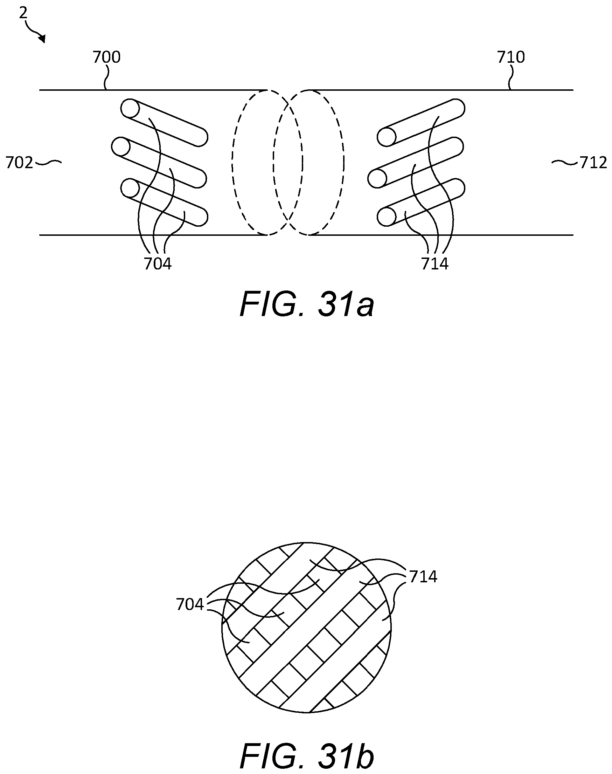

Another possible embodiment is shown in Figures 31a and 31b, and comprises multiple cross bars 704, 714 that are diagonally arranged as both the first part of the flow modifying arrangement, displaced within the flow path 702 of the first connector 700, and as the second part of the flow modifying arrangement, displaced within the second path 712 of the second connector 710. The cross bars in either of the first and second parts are arranged substantially diagonally opposite, so that when the flow modifying arrangement interacts, a mesh like restriction is formed, as shown in the front on cross-sectional view of the connector assembly shown in Figure 31b. The mesh like restriction acts to alter the resistance to flow in the gas flow path of the connector assembly. The thickness of the cross bars can be altered to change the size of the flow restriction. In similar embodiments, the bar(s) of the first or second part of the flow modifying arrangement extend(s) substantially transverse to the gas flow path in a different orientation compared to the bar(s) of the other of the first or second part of the flow modifying arrangement.

In further embodiments, the dimensions and/or shape of one or more bar(s), or blocking members and/or the dimensions and/or shape of an orifice or venturi is selected to provide a desired change in a flow property across the flow modifying arrangement.

As such, the different features of the first and/or second parts of the flow modifying arrangement are not limited to the examples shown, they can be used in any combination. For example, the features of the one or more bar and/or blocking member, including the shape, thickness, and orientation within the flow path may be selected to provide a desired change in a flow property across the flow modifying arrangement. Similarly, the features or characteristics of the orifice and/or venturi, including the shape or curve of the venturi, the position of the throat of the venturi, the shape of the orifice, and the protrusion shapes and/or orientations to form an orifice, may be selected to provide a desired change in a flow property across the flow modifying arrangement.

Furthermore, the axial location of the bar or bars, the orifice, the blocking member and/or the curved geometries of the venturi may also be varied. The axial distance between the individual features, and the first and second parts of the flow modifying arrangement may also be varied.

Additionally, or alternatively, the features of the embodiments noted as being used as the first part of the flow modifying arrangement may be used as or in the second part of the flow modifying arrangement, and vice versa. For example, in embodiments described as having a bar and a planar venturi, the bar may be in the first part of the flow modifying arrangement, or the second part, and vice versa for the planar venturi. As such, there can be many combinations of restriction members employed in either of the two parts that will provide a desired change in a flow property across the flow modifying arrangement, such as altering the resistance to flow within the gas flow path, when the flow modifying arrangement is formed.

### Tube assembly

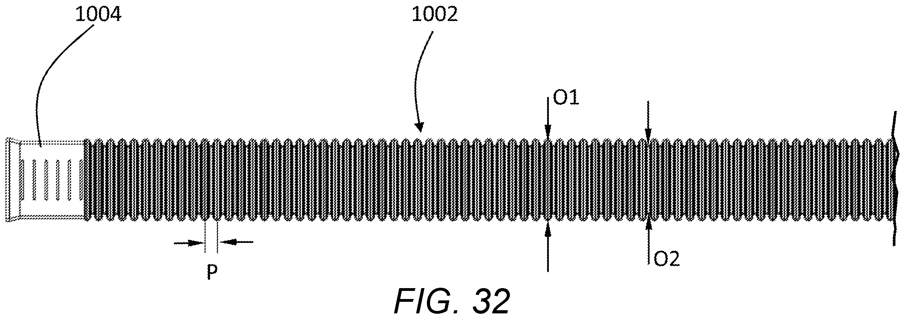

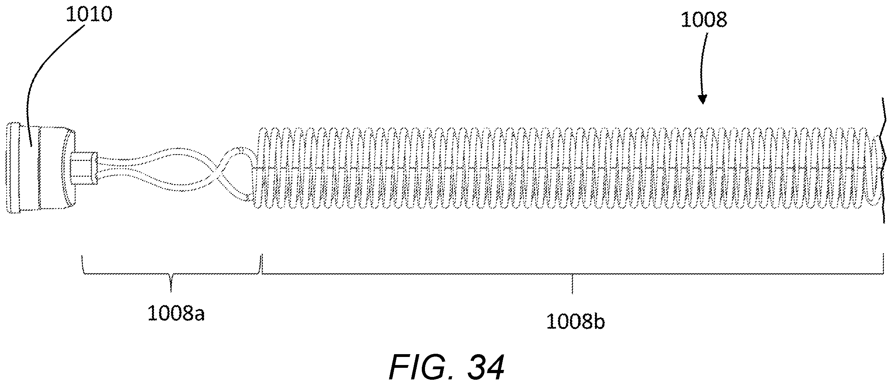

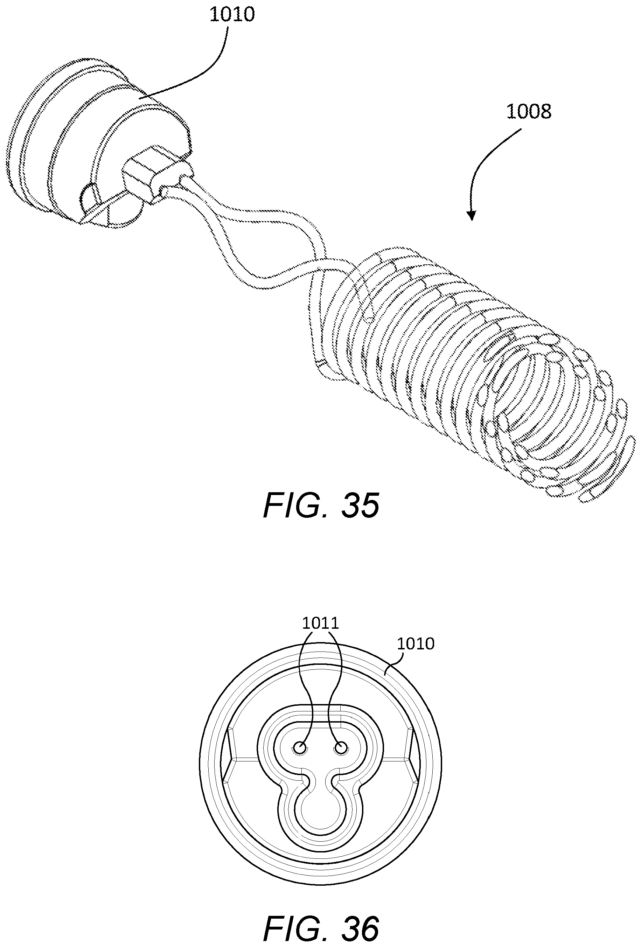

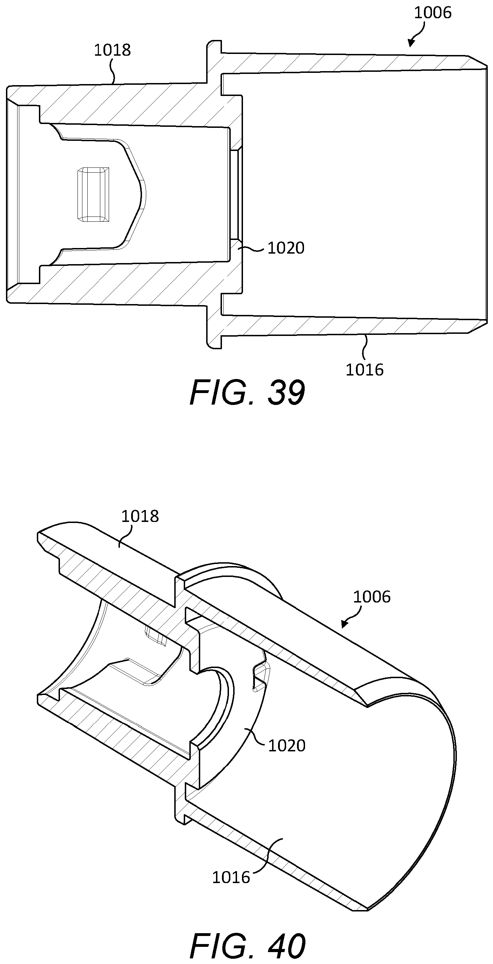

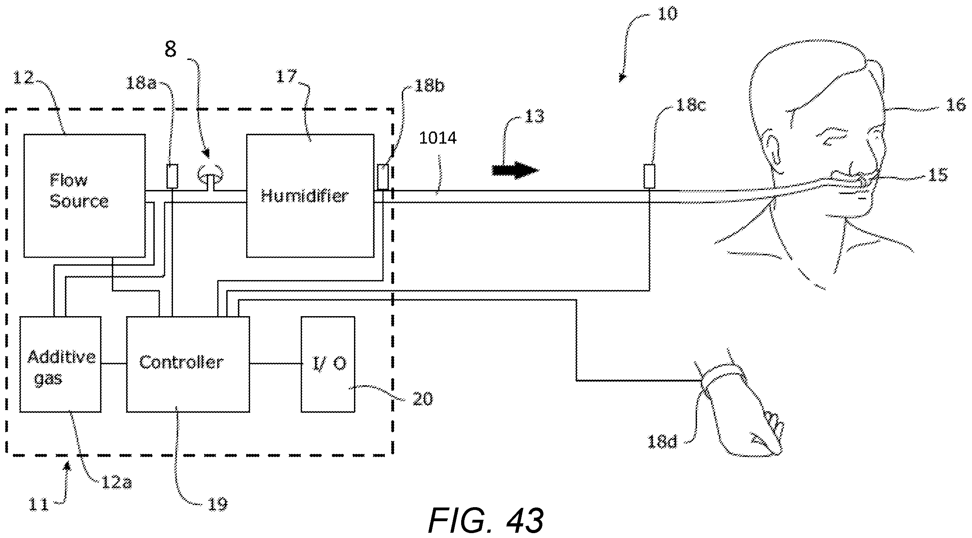

Figures 32 to 40 illustrate a tube assembly 1014 for use in a respiratory system such as the one illustrated in Figure 43. The respiratory system comprises a flow source 12, and a respiratory circuit comprising one or more of a pressure relief valve 8, the tube assembly 1014 in fluid communication with the flow source 12, and optionally a chamber of the humidifier 17 and patient interface 15.

The respiratory circuit has a resistance to gas flow downstream of the pressure relief valve that is predetermined, to be compatible with correct operation of the pressure relief valve 8. To provide the required resistance to flow for the respiratory circuit, one or more components of the respiratory circuit may be configured and/or provide one or more features to alter resistance to gases flow through the circuit. In one embodiment, the tube assembly has a resistance to gas flow of more than to zero cmH2O to about 30 cm H2O at a selected gas flow rate. The respiratory circuit may have a resistance to gas flow of 30 - 50 cmH2O at a gas flow rate of 70 L/min, more preferably about 40cmH2O at a gas flow rate of 70 L/min.

In some embodiments, the feature(s) to alter resistance may include a flow disrupting feature such as a flow obstruction or a construction provided along the flow path through the respiratory circuit. Various form flow obstruction or constructions are envisaged such as an orifice, a venturi, or a flow obstruction such as those discussed above in relation to the connection assembly.

The flow disrupting feature may be provided in any one or more of the components of the respiratory system. In preferred embodiments, the flow disrupting feature is provided in the inspiratory conduit tube assembly. The flow disrupting feature may be provided along the tube assembly conduit or by an end connector of the tube assembly.

The high gas flow may be dry or humidified. The resistance to flow may be impacted by the humidity of the gas flow, with resistance to flow generally being higher with increased humidity. In some configurations the gas flow may be humidified to contain greater than 10 mg/L of water, or greater than 20 mg/L, or greater than 30 mg/L, or up to 44 mg/L. In some configurations the gas flow may be heated to between 21 °C to 42 °C, or between 25 °C to 40 °C, or between 31 °C to 37 °C, or about 31 °C, or about 37 °C.

The resistance to flow of the exemplary tube assembly illustrated herein, may be between about 0.5 and about 25 cmH2O for a dry, non-humidified gases flow at a flow rate of between about 20 L/min and about 90 L/min. For a humidified flow at a flow rate of between about 20 L/min and about 90 L/min, the resistance to flow of the tube assembly may increase to be between about 0.5 and about 28 cmH2O.

The resistance to flow of the exemplary tube assembly illustrated herein, may be between about 0.5 and about 1.5 cmH2O, preferably about 1 cmH2O for both humidified and non-humidified gases flow at a flow rate of 20 L/min.

The resistance to flow of the exemplary tube assembly illustrated herein, may be between about 2 and about 3 cmH2O, preferably about 2.5 cmH2O for a dry, non-humidified gases flow at a flow rate of 30 L/min. For a humidified flow at 30 L/min, the resistance to flow of the tube assembly may increase to be between about 2 and about 3.5 cmH2O, preferably about 3 cmH2O.

The resistance to flow of the exemplary tube assembly illustrated herein, may be between about 3.5 and about 5.5 cmH2O, preferably about 4.5 cmH2O for a dry, non-humidified gases flow at a flow rate of 40 L/min. For a humidified flow at 40 L/min, the resistance to flow of the tube assembly may increase to be between about 4 and about 6 cmH2O, preferably about 5 cmH2O.

The resistance to flow of the exemplary tube assembly illustrated herein, may be between about 11 and about 16 cmH2O, preferably about 13 cmH2O for a dry, non-humidified gases flow at a flow rate of 70 L/min. For a humidified flow at 70 L/min, the resistance to flow of the tube assembly may increase to be between about 12 and about 17 cmH2O, preferably about 15 cmH2O.

The resistance to flow of the exemplary tube assembly illustrated herein, may be between about 18 and about 25 cmH2O, preferably about 21 cmH2O for a dry, non-humidified gases flow at a flow rate of 90 L/min. For a humidified flow at 90 L/min, the resistance to flow of the tube assembly may increase to be between about 20 and about 28 cmH2O, preferably about 24 cmH2O.

The tube assembly may form the inspiratory conduit, or other conduits in the respiratory circuit such as a conduit upstream of the humidifier. Features of the tube assembly may be selected to provide the desired resistance to flow through the circuit. For example, the dimensions of the bore of the conduit and connector, the shape of the walls of the conduit, the length of the conduit, and/or the shape or form of any heater wires positioned in the gases flow path, within the conduit bore. Alternatively, for some respiratory systems, the length of the conduit may be increased to increase resistance to flow, or decreased to reduce resistance to flow.

For a conduit with a corrugated wall, the minor and major dimensions of the corrugations, the corrugation pitch, and/or the surface roughness of the internal conduit surface may be selected to provide a desired RTF. For the corrugated wall, the minor inner dimension corresponds to the inner diameter at a corrugation 'trough', that is, the smallest inner diameter along a corrugation; the major inner dimension corresponds to the inner diameter at a corrugation 'peak, that is, the largest inner diameter along a corrugation. Likewise, for the corrugated wall, the minor outer dimension corresponds to the outer diameter at a corrugation 'trough', that is, the smallest outer diameter along a corrugation; the major outer dimension corresponds to the outer diameter at a corrugation 'peak, that is, the largest outer diameter along a corrugation.

In some embodiments, a heating wire may be provided in the inspiratory conduit to prevent condensate forming from the flow of humidified gasses through the conduit. The heating wire may be provided in the wall of the conduit or external to the conduit to have no impact on the resistance to flow through the conduit. Alternatively, the heating wire may be provided in the flow path of the conduit. When in the flow path of the conduit, the heating wire may alter or increase the resistance to flow.

In further embodiment, a heating wire clip is provided in the gas flow path. The heating wire clip may maintain the heating wire in place. The heating wire clip may also alter or increase the resistance to flow when in the flow path of the conduit.

The shape and length of the heating wire may be selected to provide the desired resistance to flow. For example, in some embodiments the heating wire is wound in a helical manner along a major part of the length of the conduit, however, to increase resistance to flow, the heating wire may be wound back over itself, a double helical manner. The length of the unwound portion of the heating wire adjacent electrical connectors for the wire may also be modified to alter the RTF.

Two or more components of the respiratory circuit may be provided together as a kit of parts for assembly by a user. Such a kit of parts would typically contain two or more of the inspiratory conduit or tube assembly, a chamber for a humidifier, a conduit for use upstream of the humidifier, a patient interface, and/or a filter.

Figures 32 to 40 illustrate one example tube assembly 1014 configured to provide an increased resistance to flow therethrough. This exemplary tube assembly is configured for use in a high flow respiratory system preferably delivering a gas flow having a flow rate of between 20 and 90L/min to a patient. The high flow respiratory system also preferably comprises a flow compensated pressure relief valve that is configured to preferably operate between about 20 and about 90 litres per minute. The pressure relief valve may comprise a sensing member to sense flow rate and is configured to dynamically change a pressure threshold of the pressure relief valve in use.

The tube assembly 1014 is for use in a respiratory circuit that further comprises a humidification chamber and a conduit upstream of the humidifier for fluidly connecting between the flow source and the humidification chamber. The respiratory circuit has a total resistance to gas flow of 30 - 50 cmH2O at a gas flow rate of 70 L/min, more preferably 35 - 45 cmH2O at a gas flow rate of 70 L/min, more preferably about 40 cmH2O at a gas flow rate of 70 L/min.

The tube assembly 1014 comprises an inspiratory conduit 1002 about 2.4m long and having a corrugated wall. The corrugations have an outer diameter of 23.6 mm (minor, O2) and 24.4 mm (major, O1), and an inner diameter of 20.1 mm (minor) and 20.6 mm (major), and a pitch, P of 4.5 mm. However, in other embodiments (with or without corrugations), it is envisaged that these dimensions will vary. For example, the inner diameter may be between about 10 and 30 mm, preferably between about 15 and 25 mm, more preferably 19 - 25 mm. For embodiments having corrugations, the minor inner diameter is most preferably between 19 - 22 mm and the major inner diameter is most preferably between 23 - 25 mm. The pitch P may be between 3 to 5 mm, or more preferably 3.5mm or 4.5mm.

The length of the conduit may also vary, for example, the length may be between 1 m and 3 m, preferably between 1.5 m and 2.5 m.

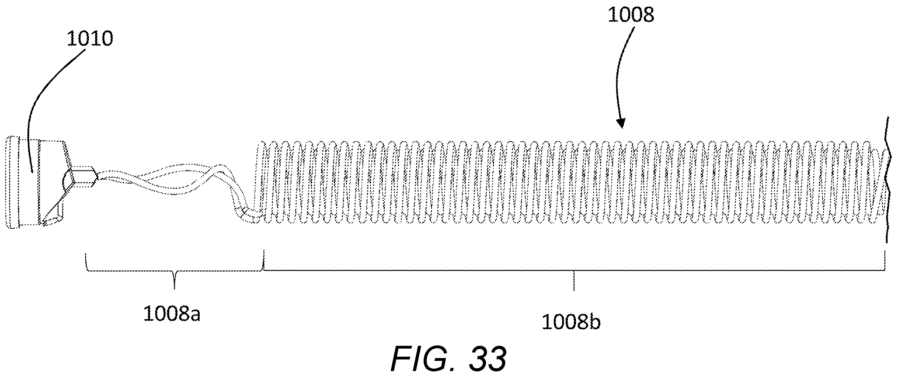

A heater wire 1008, shown in Figures 33 to 35, is provided in the tube assembly 1014. The heating wire is provided within the inspiratory conduit 1002 and extends substantially along the length of the inspiratory conduit. The heater wire comprises a coiled portion 1008b and a tail portion 1008a, the ends of which are overmoulded to a connector 1010 such that electrical contacts 1011 are provided on the connector for coupling the heater wire 1008 to an electrical source.

In this exemplary embodiment, the coil portion 1008b of the heater wire is wound in the form of a double helix with a maximum outer diameter of about 21.0 mm. The length of the coil tail portion 1008a is between about 30 mm and about 65 mm. The heating wire has an electrical resistance of about 11 to 13 ohm/metre, preferably 11.4 to about 12.7 ohm/metre at room temperature. However, other embodiments of the heating wire are envisaged.

A tube connector 1006 is provided at one end of the tube assembly 1014. The tube connector 1006 may also be provided at the other end of the tube assembly 1014. The tube connector 1006 has a cuff portion 1016 for receiving an end of the inspiratory conduit 1004, for example by way of a press fit. The connector may be permanently attached to the inspiratory conduit 1002.

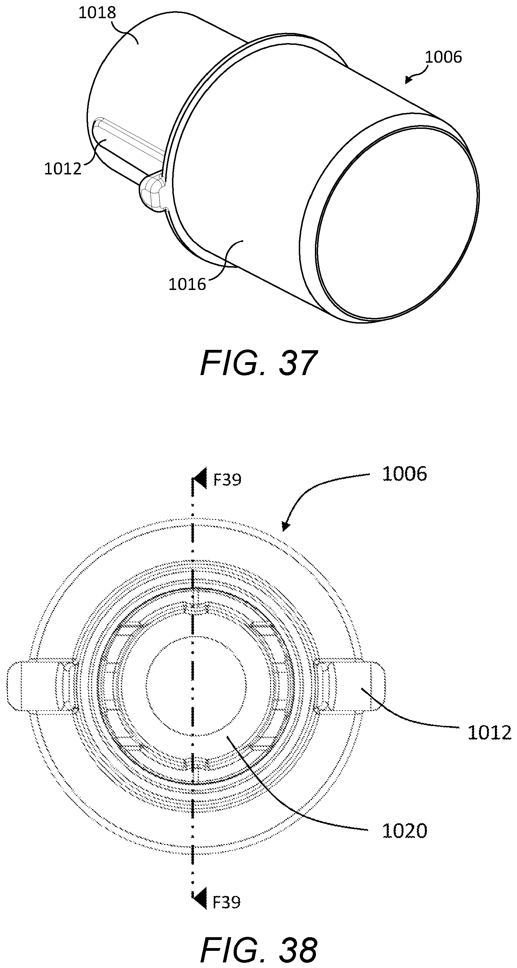

The tube connector 1006 has an attachment portion 1018 (Figures 37-40) for coupling the tube assembly to another component in the respiratory circuit. The attachment portion 1018 may comprise alignment features to assist with orientating and coupling the tube assembly to another component in the respiratory circuit. The alignment features may be internal or external. The alignment features may comprise a guide, such as a channel or a stop, or may include visual markings for a user to align when assembling the circuit. In the embodiment shown, the alignment features comprise external guide ribs 1012.

A flow disrupting feature in the form of an orifice 1020 is provided in the connector 1004, in the flow path. Alternatively, the flow disrupting feature in the form of an orifice 1020 may be provided anywhere else in the tube assembly or respiratory circuit. In this embodiment, the orifice comprises an orifice plate that is integral with the walls of the connector. The orifice defines an opening with a diameter of between about 5 mm and about 10 mm, preferably between about 6 mm and about 8 mm, more preferably between about 7.0 mm and about 7.5 mm, for example about 7mm. For embodiments with a non-circular orifice, the orifice may define an opening with a cross sectional area of between about 20 mm² and about 79 mm², preferably between about 28 mm² and about 50 mm², for example about 38.5 mm².

This example tube assembly embodiment, including the assembled connector, inspiratory conduit, and heater wire, may provide a combined resistance to gas flow when humidified of 12 - 17cmH2O at a gas flow rate of 70 L/min, more preferably about 15 cmH2O at a gas flow rate of 70 L/min.

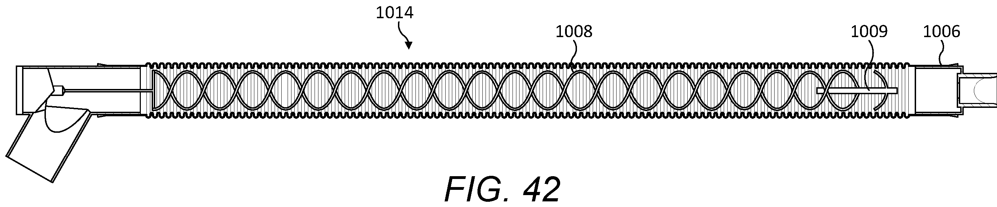

Furthermore, as shown in Figure 42, a heating wire clip 1009 may be provided in the gas flow path of the tube assembly 1014. The heating wire clip 1009 may maintain the heating wire 1008 in place. The heating wire clip may also impact and may increase the resistance to flow.

Certain features, aspects and advantages of the embodiments described above may be used for providing gases to and/or removing gases from a patient, such as in high flow respiratory support, positive airway pressure (PAP), respirator, anaesthesia, ventilator, and/or insufflation systems.

Preferred embodiments of the invention have been described by way of example only and modifications may be made thereto without departing from the scope of the invention.

## Claims

1. A connector assembly (2) for use in a respiratory support system, comprising:
a first connector (4, 100, 200, 300, 400);
a second connector (6, 110, 210, 310, 410) for connection to the first connector (4, 100, 200, 300, 400) to thereby define a gas flow path through the connectors (4, 100, 200, 300, 400, 6, 110, 210, 310, 410); and
a flow modifying arrangement having a first part (104, 204, 304, 404) and a second part (114, 214, 314, 414);
wherein the first connector (4, 100, 200, 300, 400) comprises the first part (104, 204, 304, 404) of the flow modifying arrangement, and the second connector (6, 110, 210, 310, 410) comprises the second part (114, 214, 314, 414) of the flow modifying arrangement; and wherein the flow modifying arrangement is configured to change a property of a gas flow along the gas flow path (120, 320) when the first and second connectors (4, 100, 200, 300, 400, 6, 110, 210, 310, 410) are connected,
wherein the first part (104, 204, 304, 404) of the flow modifying arrangement and the second part (114, 214, 314, 414) of the flow modifying arrangement together define a tortuous flow path through the flow modifying arrangement.

2. The connector assembly of claim 1, wherein the property of the gas flow along the gas flow path (120) is one or more of: pressure, turbulence, and velocity.

3. The connector assembly of claim 1 or 2, wherein the first part (104, 204, 304, 404) of the flow modifying arrangement and the second part (114, 214, 314, 414) of the flow modifying arrangement together define a high resistance portion of the gas flow path (120) having a higher resistance to flow than portions of the gas flow path (120) immediately upstream and downstream of the flow modifying arrangement, and wherein the high resistance portion forms a dominant flow restriction in the respiratory system.

4. The connector assembly of claim 3, wherein the high resistance portion of the gas flow path (120) comprises a cross sectional area that is selected to provide a pre-determined change to a property of gas flow across the flow modifying arrangement.

5. The connector assembly of any one of the preceding claims, wherein the first part (104, 204, 304, 4044) of the flow modifying arrangement and the second part (114, 214, 314, 414) of the flow modifying arrangement are arranged co-axially.

6. The connector assembly of any one of the preceding claims, wherein the first part (104, 204, 304, 404) of the flow modifying arrangement comprises one or more of: a constriction or an obstruction, orifice, venturi, and a blocking member (304); and wherein the second part (114, 214, 314, 414) of the flow modifying arrangement comprises one or more of: a constriction or an obstruction, orifice, venturi, and a blocking member.

7. The connector assembly of any one of the preceding claims, wherein the first part (104, 204, 304, 404) of the flow modifying arrangement comprises at least one blocking member (304) that is substantially cylindrical, frustoconical, cuboidal, pyramidal, prismatic, conical, or spherical.

8. The connector assembly of claim 7, wherein the blocking member (304) comprises a longitudinal axis that is substantially parallel to a longitudinal axis of the first connector.

9. The connector assembly of any one of the preceding claims, wherein the first part (104, 204, 304, 404) of the flow modifying arrangement comprises one or more bars.

10. The connector assembly of any one of the preceding claims, wherein the one or more bars extends across at least a portion of the gas flow path (120).

11. The connector assembly of any one of the preceding claims, wherein the first part (104, 204, 304, 404) of the flow modifying arrangement and the second part (114, 214, 314, 414) of the flow modifying arrangement do not substantially alter a property of a gas flow across the first connector (4, 100, 200, 300, 400), when the first connector (4, 100, 200, 300, 400) is separate from the second connector (6, 110, 210, 310, 410).

12. The connector assembly of any one of the preceding claims, wherein the first connector (4, 100, 200, 300, 400) is releasably engageable with the second connector (6, 110, 210, 310, 410).

13. The connector assembly of any one of the preceding claims, wherein the first connector (4, 100, 200, 300, 400) and the second connector (6, 110, 210, 310, 410) comprise alignment features to orientate the first part (104, 204, 304, 404) of the flow modifying arrangement relative to the second part (114, 214, 314, 414) of the flow modifying arrangement.

14. The connector assembly of any one of the preceding claims, comprising one or both of:
the second connector (6, 110, 210, 310, 410) being configured for coupling to a patient interface conduit or filter; and
the first connector (4, 100, 200, 300, 400) being configured for coupling to an inspiratory conduit (14) or filter.

15. The connector assembly of any one of the preceding claims, wherein the flow modifying arrangement is configured for use at flow rates of 20-90L/min, or 40-70L/min, or more than 20 L/min.

## Patentansprüche

1. Verbinderanordnung (2) zur Verwendung in einem Beatmungsunterstützungssystem, umfassend:
einen ersten Verbinder (4, 100, 200, 300, 400);
einen zweiten Verbinder (6, 110, 210, 310, 410) zum Verbinden mit dem ersten Verbinder (4, 100, 200, 300, 400), um dadurch einen Gasströmungspfad durch die Verbinder (4, 100, 200, 300, 400, 6, 110, 210, 310, 410) zu definieren; und
eine Durchflussmodifizierungsanordnung mit einem ersten Teil (104, 204, 304, 404) und einem zweiten Teil (114, 214, 314, 414);
wobei der erste Verbinder (4, 100, 200, 300, 400) den ersten Teil (104, 204, 304, 404) der Durchflussmodifizierungsanordnung umfasst und der zweite Verbinder (6, 110, 210, 310, 410) den zweiten Teil (114, 214, 314, 414) der Durchflussmodifizierungsanordnung umfasst; und wobei die Durchflussmodifizierungsanordnung so konfiguriert ist, dass sie eine Eigenschaft eines Gasstroms entlang des Gasströmungspfades (120, 320) ändert, wenn der erste und der zweite Verbinder (4, 100, 200, 300, 400, 6, 110, 210, 310, 410) verbunden sind,
wobei der erste Teil (104, 204, 304, 404) der Durchflussmodifizierungsanordnung und der zweite Teil (114, 214, 314, 414) der Durchflussmodifizierungsanordnung zusammen einen gewundenen Strömungspfad durch die Durchflussmodifizierungsanordnung definieren.

2. Verbinderanordnung nach Anspruch 1, wobei die Eigenschaft des Gasstroms entlang des Gasströmungspfades (120) eine oder mehrere der Folgenden ist: Druck, Turbulenz und Geschwindigkeit.

3. Verbinderanordnung nach Anspruch 1 oder 2, wobei der erste Teil (104, 204, 304, 404) der Durchflussmodifizierungsanordnung und der zweite Teil (114, 214, 314, 414) der Durchflussmodifizierungsanordnung zusammen einen Abschnitt mit hohem Widerstand des Gasströmungspfades (120) bilden, der einen höheren Strömungswiderstand aufweist als Abschnitte des Gasströmungspfades (120), die unmittelbar stromaufwärts und stromabwärts der Durchflussmodifizierungsanordnung liegen, und wobei der Abschnitt mit hohem Widerstand eine dominante Strömungsbehinderung im Atmungssystem darstellt.

4. Verbinderanordnung nach Anspruch 3, wobei der Abschnitt mit hohem Widerstand des Gasströmungspfades (120) eine Querschnittsfläche umfasst, die so ausgewählt ist, dass sie eine vorbestimmte Änderung einer Eigenschaft des Gasstroms über die Durchflussmodifizierungsanordnung hinweg bereitstellt.

5. Verbinderanordnung nach einem der vorstehenden Ansprüche, wobei der erste Teil (104, 204, 304, 4044) der Durchflussmodifizierungsanordnung und der zweite Teil (114, 214, 314, 414) der Durchflussmodifizierungsanordnung koaxial angeordnet sind.

6. Verbinderanordnung nach einem der vorstehenden Ansprüche, wobei der erste Teil (104, 204, 304, 404) der Durchflussmodifizierungsanordnung eines oder mehrere der Folgenden umfasst: eine Verengung oder ein Hindernis, eine Öffnung, ein Venturi-Rohr und ein Blockierelement (304); und wobei der zweite Teil (114, 214, 314, 414) der Durchflussmodifizierungsanordnung eines oder mehrere der Folgenden umfasst: eine Verengung oder ein Hindernis, eine Öffnung, ein Venturi-Rohr und ein Blockierelement.

7. Verbinderanordnung nach einem der vorstehenden Ansprüche, wobei der erste Teil (104, 204, 304, 404) der Durchflussmodifizierungsanordnung mindestens ein Blockierelement (304) umfasst, das im Wesentlichen zylindrisch, kegelstumpfförmig, quaderförmig, pyramidenförmig, prismatisch, konisch oder kugelförmig ist.

8. Verbinderanordnung nach Anspruch 7, wobei das Blockierelement (304) eine Längsachse aufweist, die im Wesentlichen parallel zu einer Längsachse des ersten Verbinders verläuft.

9. Verbinderanordnung nach einem der vorstehenden Ansprüche, wobei der erste Teil (104, 204, 304, 404) der Durchflussmodifizierungsanordnung einen oder mehrere Stäbe umfasst.

10. Verbinderanordnung nach einem der vorstehenden Ansprüche, wobei sich der eine oder die mehreren Stäbe über mindestens einen Abschnitt des Gasströmungspfades (120) erstrecken.

11. Verbinderanordnung nach einem der vorstehenden Ansprüche, wobei der erste Teil (104, 204, 304, 404) der Durchflussmodifizierungsanordnung und der zweite Teil (114, 214, 314, 414) der Durchflussmodifizierungsanordnung eine Eigenschaft eines Gasstroms über den ersten Verbinder (4, 100, 200, 300, 400) nicht wesentlich verändern, wenn der erste Verbinder (4, 100, 200, 300, 400) vom zweiten Verbinder (6, 110, 210, 310, 410) getrennt ist.

12. Verbinderanordnung nach einem der vorstehenden Ansprüche, wobei der erste Verbinder (4, 100, 200, 300, 400) mit dem zweiten Verbinder (6, 110, 210, 310, 410) lösbar in Eingriff gebracht werden kann.

13. Verbinderanordnung nach einem der vorstehenden Ansprüche, wobei der erste Verbinder (4, 100, 200, 300, 400) und der zweite Verbinder (6, 110, 210, 310, 410) Ausrichtungselemente umfassen, um den ersten Teil (104, 204, 304, 404) der Durchflussmodifizierungsanordnung relativ zum zweiten Teil (114, 214, 314, 414) der Durchflussmodifizierungsanordnung auszurichten.

14. Verbinderanordnung nach einem der vorstehenden Ansprüche, umfassend eines oder beide von:
dem zweiten Verbinder (6, 110, 210, 310, 410), der zur Kopplung mit einer Patientenschnittstellenleitung oder einem Patientenschnittstellenfilter konfiguriert ist; und
dem ersten Verbinder (4, 100, 200, 300, 400), der zur Kopplung mit einem Inspirationsschlauch (14) oder einem Inspirationsfilter konfiguriert ist.

15. Verbinderanordnung nach einem der vorstehenden Ansprüche, wobei die Durchflussmodifizierungsanordnung für die Verwendung bei Durchflussraten von 20-90L/min oder 40-70L/min oder mehr als 20 L/min konfiguriert ist.

## Revendications

1. Ensemble de connecteurs (2) destiné à être utilisé dans un système d'assistance respiratoire, comprenant :
un premier connecteur (4, 100, 200, 300, 400) ;
un second connecteur (6, 110, 210, 310, 410) destiné à être raccordé au premier connecteur (4, 100, 200, 300, 400) afin de définir ainsi un chemin d'écoulement de gaz à travers les connecteurs (4, 100, 200, 300, 400, 6, 110, 210, 310, 410) ; et
un agencement de modification d'écoulement présentant une première partie (104, 204, 304, 404) et une seconde partie (114, 214, 314, 414) ;
dans lequel le premier connecteur (4, 100, 200, 300, 400) comprend la première partie (104, 204, 304, 404) de l'agencement de modification d'écoulement, et le second connecteur (6, 110, 210, 310, 410) comprend la seconde partie (114, 214, 314, 414) de l'agencement de modification d'écoulement ; et dans lequel l'agencement de modification d'écoulement est configuré pour modifier une propriété d'un écoulement de gaz le long du chemin d'écoulement de gaz (120, 320) lorsque les premier et second connecteurs (4, 100, 200, 300, 400, 6, 110, 210, 310, 410) sont raccordés,
dans lequel la première partie (104, 204, 304, 404) de l'agencement de modification d'écoulement et la seconde partie (114, 214, 314, 414) de l'agencement de modification d'écoulement définissent ensemble un chemin d'écoulement tortueux à travers l'agencement de modification d'écoulement.

2. Ensemble de connecteurs selon la revendication 1, dans lequel la propriété de l'écoulement de gaz le long du chemin d'écoulement de gaz (120) est une ou plusieurs éléments parmi : la pression, la turbulence et la vitesse.

3. Ensemble de connecteurs selon la revendication 1 ou 2, dans lequel la première partie (104, 204, 304, 404) de l'agencement de modification d'écoulement et la seconde partie (114, 214, 314, 414) de l'agencement de modification d'écoulement définissent ensemble une portion à haute résistance du chemin d'écoulement de gaz (120) présentant une résistance à l'écoulement plus élevée que les portions du chemin d'écoulement de gaz (120) immédiatement en amont et en aval de l'agencement de modification d'écoulement, et dans lequel la portion à haute résistance forme une restriction d'écoulement dominante dans le système respiratoire.

4. Ensemble de connecteurs selon la revendication 3, dans lequel la portion à haute résistance du chemin d'écoulement de gaz (120) comprend une zone de coupe transversale qui est choisie pour fournir un changement prédéterminé d'une propriété d'écoulement de gaz à travers l'agencement de modification d'écoulement.

5. Ensemble de connecteurs selon l'une quelconque des revendications précédentes, dans lequel la première partie (104, 204, 304, 4044) de l'agencement de modification d'écoulement et la seconde partie (114, 214, 314, 414) de l'agencement de modification d'écoulement sont agencées de manière coaxiale.

6. Ensemble de connecteurs selon l'une quelconque des revendications précédentes, dans lequel la première partie (104, 204, 304, 404) de l'agencement de modification d'écoulement comprend un ou plusieurs éléments parmi : un rétrécissement ou une obstruction, un orifice, un venturi et un élément de blocage (304) ; et dans lequel la seconde partie (114, 214, 314, 414) de l'agencement de modification d'écoulement comprend un ou plusieurs éléments parmi : un rétrécissement ou une obstruction, un orifice, un venturi et un élément de blocage.

7. Ensemble de connecteurs selon l'une quelconque des revendications précédentes, dans lequel la première partie (104, 204, 304, 404) de l'agencement de modification d'écoulement comprend au moins un élément de blocage (304) qui est sensiblement cylindrique, tronconique, cuboïde, pyramidal, prismatique, conique ou sphérique.

8. Ensemble de connecteurs selon la revendication 7, dans lequel l'élément de blocage (304) comprend un axe longitudinal sensiblement parallèle à un axe longitudinal du premier connecteur.

9. Ensemble de connecteurs selon l'une quelconque des revendications précédentes, dans lequel la première partie (104, 204, 304, 404) de l'agencement de modification d'écoulement comprend une ou plusieurs barres.

10. Ensemble de connecteurs selon l'une quelconque des revendications précédentes, dans lequel les une ou plusieurs barres s'étendent sur au moins une portion du chemin d'écoulement de gaz (120).

11. Ensemble de connecteurs selon l'une quelconque des revendications précédentes, dans lequel la première partie (104, 204, 304, 404) de l'agencement de modification d'écoulement et la seconde partie (114, 214, 314, 414) de l'agencement de modification d'écoulement ne modifient pas sensiblement une propriété d'un écoulement de gaz à travers le premier connecteur (4, 100, 200, 300, 400), lorsque le premier connecteur (4, 100, 200, 300, 400) est séparé du second connecteur (6, 110, 210, 310, 410).

12. Ensemble de connecteurs selon l'une quelconque des revendications précédentes, dans lequel le premier connecteur (4, 100, 200, 300, 400) peut être mis en prise de manière amovible avec le second connecteur (6, 110, 210, 310, 410).

13. Ensemble de connecteurs selon l'une quelconque des revendications précédentes, dans lequel le premier connecteur (4, 100, 200, 300, 400) et le second connecteur (6, 110, 210, 310, 410) comprennent des caractéristiques d'alignement pour orienter la première partie (104, 204, 304, 404) de l'agencement de modification d'écoulement par rapport à la seconde partie (114, 214, 314, 414) de l'agencement de modification d'écoulement.

14. Ensemble de connecteurs selon l'une quelconque des revendications précédentes, comprenant l'un ou les deux éléments parmi :
le second connecteur (6, 110, 210, 310, 410) étant configuré pour être couplé à un conduit ou un filtre d'interface patient ; et
le premier connecteur (4, 100, 200, 300, 400) étant configuré pour être couplé à un conduit inspiratoire (14) ou à un filtre.

15. Ensemble de connecteurs selon l'une quelconque des revendications précédentes, dans lequel l'agencement de modification d'écoulement est configuré pour être utilisé à des débits allant de 20 à 90 l/min, ou de 40 à 70 l/min, ou supérieurs à 20 l/min.
